(19) Europäisches Patentamt
European Patent Office
Office européen des brevets

(11) **EP 0 673 653 B1**

(12) **EUROPEAN PATENT SPECIFICATION**

(45) Date of publication and mention
of the grant of the patent:
**09.05.2007 Bulletin 2007/19**

(51) Int Cl.:
*A61K 35/48* (2006.01)     *A61K 35/12* (2006.01)
*C07C 15/06* (2006.01)

(21) Application number: **94905613.9**

(22) Date of filing: **20.07.1993**

(86) International application number:
**PCT/UA1993/000004**

(87) International publication number:
**WO 1994/002105 (03.02.1994 Gazette 1994/04)**

(54) **BIOLOGICALLY ACTIVE AGENT HAVING IMMUNOMODULATING PROPERTIES, METHOD FOR ITS OBTAINING AND PHARMACEUTICAL PREPARATION BASED ON IT**

BIOLOGISCH AKTIVE SUBSTANZ MIT IMMUNMODULIERENDEN EIGENSCHAFTEN, VERFAHREN ZU IHRER GEWINNUNG UND AUS IHR HERGESTELLTE, PHARMAZEUTISCHE ZUBEREITUNG

AGENT BIOLOGIQUEMENT ACTIF AYANT DES PROPRIETES D'IMMUNOMODULATION, PROCEDE POUR SA PRODUCTION ET PREPARATION PHARMACEUTIQUE A BASE DE CET AGENT

(84) Designated Contracting States:
**AT BE CH DE FR GB GR LI NL**

(30) Priority: **21.07.1992 RU 5055549**
**17.12.1992 UA 93030289**

(43) Date of publication of application:
**27.09.1995 Bulletin 1995/39**

(73) Proprietor: **NIKOLAENKO; Alexandr, Nikolaevich**
**Kiev, 252049 (UA)**

(72) Inventor: **NIKOLAENKO; Alexandr, Nikolaevich**
**Kiev, 252049 (UA)**

(74) Representative: **Patentanwälte**
**Zellentin & Partner**
**Rubensstrasse 30**
**67061 Ludwigshafen (DE)**

(56) References cited:
EP-A- 0 318 030       FR-A- 2 242 109
FR-A- 2 317 793       FR-A- 2 451 193
FR-A- 2 469 926       US-A- 3 522 348
US-A- 4 169 139       US-A- 4 810 495

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**EP 0 673 653 B1**

**Description**

The group of inventions relates to the field of biologically active agents with immunomodulating action for veterinary and medical usage aimed at the normalization of the physiological state in human and animal organisms, including regeneration of functional activity of organs and tissues.

In accordance with this invention, a biologically active agent is referred to substances of animal origin and includes organic compounds.

Various pathological states in animal and human organisms cause parameters of their physiological balance to deviate from the norm. In these cases, curative influence on the organism, including prophylaxis, means stabilization, that is maintenance of physiological parameters in the norm.

The group of inventions deals with a biologically active agent, a method of its production and a preparation based on it.

The normalization of the physiological state of human and animals can be achieved in two directions: firstly, by means of direct influence on pathology and secondly, by means of indirect influence on systems, organs and tissues of the organism, which are responsible for the parameters of homeostasis to be maintained in the norm.

The administration of hormonal preparations, mediators, growth factors, chemotherapeutic drugs and similar preparations provides direct influence on target cells by means of reception. For example, in the case of treatment of diabetes mellitus administered insulin compensates functional insufficiency of the pancreas. In the case of disorders in functional activity of the nervous system neuromediators are to be administered because of their ability to normalize the threshold sensitivity of neurones, and consequently improve the transmission of neural impulses. In the case of disorders in functional activity of the immune system immunomediators, which are able to correct immune response, should be used.

In the case of mediated influence on the organism the treatment of diseases is achieved by means of mobilization of organism internal reserves. For instance, in the processes connected with regeneration of tissues and organs, their rehabilitation and recovery of their functions, as well as in the cases of inflammatory and infectious processes, it is necessary to stimulate the immune system of the organism. Owing to this stimulation, an improvement in immunological as well as general state of the organism may be achieved.

There is a known biologically active agent on the basis of embryonic tissue, which has immunomodulating activity and is able to restore the physiological state of the organism and regulate the cellular metabolism (FR-A-2413912). This biologically active agent (herein after BAA) is prepared on the basis of ingredients of disintegrated and homogenized animal embryonic tissues in a mixture with a physiological solution.

Due to the presence of an immunomodulating factor in its composition, a BAA has a comparatively high immunomodulating activity, thereby ensuring the normalization of the physiological state of the organism, and resulting in an improvement of animal productivity by 5-15% in comparison with control.

However, the effectiveness of this known BAA is insufficient so far as it includes, besides the immunomodulating factors, also immunodepressants which prevent full realization of its immunomodulating effect. As a matter of fact, the immunomodulating activity of this BAA is an integral effect ensured by the presence of immunostimulating factors and depressants at the same time. Thus, the actual specific activity of this known BA , which depends upon the ratio between immunostimulating factors and depressants, is relatively low, as a consequence of a high concentration of depressants.

Another disadvantage of this known BAA is its high content of high-molecular compounds. These compounds are immunogenes that cause various side immune reactions (allergy, delayed hypersensitivity and so on).

To a considerable extent, all disadvantages of the described BAA depend on the method of its production, where the technological process finishes with the operation of disintegration and homogenization of raw material from animal tissue, which results in the inevitable presence of a significant quantity of depressants and high-molecular compounds in its composition.

There is also another known BAA with immunomodulating activity designed for the normalization of the physiological state of the organism. This BAA is prepared on the basis of hydrolysis products from disintegrated raw materials of animal origin, taken preferably from immature animals. A method is known for its use in healing traumatized areas of the body (US-A-4455302).

This BAA is a very effective agent for the normalization of the physiological state in the organism. It could be explained by the fact that the mentioned BAA is characterized by a high ratio between the immunostimulation and immunoinhibition effects due to its high content of immunomodulating factors as a result of the presence of hydrolysis products. In addition, it contains a lowered amount of high-molecular compounds caused by the process of hydrolysis.

However, the effectiveness of this BAA is insufficient because of the presence of a considerable quantity of ballast substances and immunodepressants in its composition. Besides, this BAA can also cause side effects (allergy and so on) as a consequence of the presence of high-molecular compounds in its composition. These compounds are immunogenes and their molecular mass is more than 10.0kDa.

Another disadvantage of the known BAA is the fact that the composition of the final product is not permanent and fluctuates from one batch to another, which causes difficulties with the standardization.

2

The properties of the known BAA, and first of all its immunomodulating activity, are directly connected with the method of its production, which includes the hydrolysis of washed and disintegrated raw material of animal origin, centrifugation of the hydrolysate, filtration and collection of the desired product. It should be taken into account, that hydrolysis is to be done in a solution of a weak organic acid at pH 3.6 to 6.5 and a temperature of about 68°C. As raw material the legs of immature poultry no older than 9 weeks are used.

The desired product is produced in the form of powder or gel, which is to be applied to injured parts of the body.

The method used for the production of the desired product does not allow the elimination of immunodepressants, which significantly reduces the efficiency of the BAA produced.

It should be taken into consideration, that the water-soluble components of homogenized tissue-derived drugs are, to a great extent, stimulators of functional activity of organs and tissues, and their influence is unidirectional. So far. BAA activity depends only upon its concentration in the organism and does not depend on the physiological state of the organism. This fact requires to keep strictly to dosages of pharmacological drugs based on this BAA in the process of treatment.

The mentioned disadvantages are partially eradicated in other known BAAs whose effects develop in dependence on the physiological state of the organism. In the case of its normalization, the activity of that type BAAs ceases. Because of this, BAAs on the basis of components of cell membranes of animal tissues are of great interest, so far as they are able to respond to changes in the parameters of homeostasis of an organism (SU-A-1412596: EP-A-0106285; EP-A-0157427).

Preparations in the form of vaccines, based on said BAAS contain specific antigens which appear on the surface of the cells during neoplasia. Thereby, such vaccines activate the process of elimination of this pathology.

The efficiency 01 said preparations depends directly upon the quantity of antigens presented in the organism, and after elimination of neoplasia their action ceases, which indicates the presence of bilateral self-regulation.

Another disadvantage of these known preparations is their high specificity to initial pathology, in other words, they have a very narrow spectrum of activity in restoration (normalization) of physiological parameters. Because of this, in principle they are not able to have a wide spectrum of activity in recovery of the parameters of the physiological state of the organism.

Another disadvantage is the fact that all these vaccines are used for the elimination of pathology and only undirectly can influence the normalization of the physiological state of the organism. Besides, these known BAAs are not capable of activating the regenerative and reconstructive processes. Such BAAs have to satisfy the strict requirements in respect of the degree of antigen purity in order to avoid side effects.

All known BAAs are produced by known method, according to which a raw animal material is disintegrated and homogenized with following cell membrane isolation from the homogenate. After solubilization, components of cell membranes are produced in the form of glycoproteins (SU-A-1412596; EP-A-0106285; EP-A-0157427).

An advantage of the known method is the fact that it allows the high-specific carbohydrate-containing components of cell membranes, which are able to induce response of the immune system of organism to the presence of tumour - specific antigen, to be obtained.

The carbohydrate-containing components of cell membranes are selected with help of specific lectines capable of binding to certain terminal carbonyl groups.

However, it should be taken into consideration that during the realization of operations of the known method, cell membranes are solubilized by means of detergents, usually of non-ionic origin, for instance Triton X-100 detergent. These detergents are difficult to eliminate and they are capable of inducing side effects.

Another disadvantage of the known method is its low productivity. Besides, it is highly laborious and dependent on the availability of specific tumor tissues used as raw material.

The closest prior art agent is a BAA with immunomodulating activity on the basis of organic compounds of cell membrane components produced from a disintegrated homogenized animal tissue (EP-A-0318030).

In comparison with the above described BAAs this BAA is even more effective, being characterized by 62% increase in killer activity in respect to specific tumour cells versus control. This may be explained by the fact that this BAA based on glycoprotein is a marker in the growth of malignant cells and specifically stimulates killer activity against these cells.

But this BAA keeps all above named disadvantages, namely:

- high specificity to initial pathology (neoplasia), that is a very narrow spectrum of curative activity;
- displaying only killer activity;
- rather high level of side effects, so far as this BAA is a high - molecular compound.

AS in the described cases, the properties of this BAA, primarily its specific activity, are directly connected with the method of its production, which includes disintegration and homogenization of raw animal material followed by ultracentrifugation and isolation of sediment from homogenate. The cell membrane components are produced from the sediment by means of its resuspending and solubilizing (EP-A-0318030).

In comparison with the above mentioned methods of BAA production, this method is more effective in isolating high-specific carbohydrate-containing components of cell membranes.

However, this known method has disadvantages as well, namely:

- the necessity for the use of a detergent which is rather difficult to get rid of and whose presence cause adverse side effects:

- comparatively low productivity as well as high laboriousness;
- the dependence on the availability of specific tumor tissues as raw material.

There is a known medical preparation for the normalization of physiological state of human and animals, on the basis of organic compounds of cell membran components (EP-A-0318030). The preparation is administered for a long time as intramuscular or intravenous injections at a daily dose of 0. 001 to 1, 000 µg.

The dose depends upon age, sex and stage of illness. As excipients for intravenous injections a physiological solution of sodium chloride or buffer solutions are used.

The main active ingredient of this preparation is a BAA in the form of tumour - specific antigen that induces immune response of the organism. In order to strengthen and prolongate this response, such an antigen is incorporated in liposomes. For this purpose, various adjuvants based on oils are used as excipients for intramuscular injections. The above indicated BAA is suspended in these oils.

A disadvantage of the known pharmacological preparation is the fact, that it ensures high - specific stimulation of the immune system of an organism, which is oriented to the elimination of a concrete form of neoplasia. Such a stimulation of the immune system is followed by the manifestation of numerous adverse side effects disordering the parameters of homeostasis. Particularly, local inflammation often develops following intramuscular or subcutaneous injections, while generalized inflammation arises following intravenous injections. These reactions can be accompanied with allergy, delayed hypersensitivity and other phenomena.

There is another known preparation for the normalization of the physiological state of human and animals, which includes a biologically active agent on the basis of organic compounds of cell membrane components of animal tissues and as a second ingredient, an excipient (US-A-4455302).

A disadvantage of this preparation is the fact, that it can be only used as local applications to injured parts of the body, consequently its efficacy is comparatively low.

The noted disadvantages are partially eliminated in another known preparation with immunomodulating activity, designed for the normalization of the physiological state of animals (SU-A-904707), which is administered intramuscularly at a dose of 0.75 to 1.0 ml/kg of the body weight with 3-day intervals between injections.

A disadvantage of this preparation is its comparatively low efficiency because of the presence of immunodepressants in its composition. Besides, it can induce side effects (allergy, delayed hypersensitivity, etc.) because of the presence of immunogens in its composition.

Another disadvantage of this preparation is the presence of a lot of various biologically active substances in its composition, which causes, a danger of overdosages on one hand and the development of side effects,which makes a desired preparation more expensive, on the other hand.

There is a known method of normalization of the physiological state of human and animals suffering from diseases caused by functional insufficiency of some organs and tissues. It suggests the administration of a preparation based on organic compounds of cell membrane components of animal tissue (SU-A-904707).

This known method is of low efficacy and requires to use high doses of a BAA (up to 10 mg/kg of the body weight). This creates a danger of overdosages and development of side effects.

The noted disadvantages are eliminated by the present biologically active agent with immunomodulating activity, the present method of producing this BAA, the preparation made on its basis and its use for the manufacture of said preparation.

The analysis of the known BAAs, as well as the known methods of their production, whose total combination of features defines a technical level in this field, leads to the problem to provide a BAA with a wide spectrum of immunomodulating activity. Simultaneously, this BAA should be highly effective, free of disadvantages connected with side effects such as allergy.

This problem has not been solved in the known inventions. The solution of this problem represents the basis of the present group of inventions.

A subject matter of the present invention is a BAA with immunomodulating activity, designed for the normalization of the physiological state of the organism, including restoration of the functional activity of organs and tissues, wherein the BAA contains thermostable, low-molecular, modified components of cell membranes of homogenized animal tissue, said components being organic components, being thermostable at a temperature of up to 120°C, having a molecular mass of ≤ 10.0 kDa, and being isolated from cell membranes of cells, which have been taken from the processes of embryogenesis and/or proliferation and/or differentiation of the cells, or pathology preceding and/or accompanying the process of regeneration and/or reparation of the tissue, said agent being obtainable by disintegrating and homogenizing raw material of animal tissue, said raw material being selected from the group comprising embryonic tissue except tissue of human embryos, placental tissue, differentiating thymocytes of the thymus, spermatogones and spermatocytes of the testes, epithelial tissue of the intestine, bone marrow cells, regenerating cutaneous tissue, regenerating hepatic tissue, tissue of regenerating amphibian limbs, pathologically transformed tissue taken prior to the stage of regeneration, or any combination thereof, purifying homogenate from non-disintegrated tissue elements by means of filtration and/or centrifugation at 150 to 250 g for 10 to 15 min, isolating the sediment comprising cell membranes from said homogenate, hydrolyzing said sediment, filtering and/or centrifugating the hydrolysis products at above 1.500 g for 20 to 40 min, and collecting the supernatant of the hydrolysis products comprising cell membrane components as desired product.

Preferably, the thermostable, low-molecular components of cell membranes comprise components of plasmatic cell membranes.

Preferably, said low-molecular components of cell membranes have a molecular mass M = 0.8 - 10.0 kDa, which more preferably comprise carbohydrate-containing components.

More preferably, the low-molecular components of plasmatic cell membranes have a molecular mass M = 0.8 - 10.0 kDa, which preferably comprise carbohydrate-containing components.

The present BAA preferably contains organic compounds at the following ratio in percent by mass:

| | |
|---|---|
| Peptides | from 28.0 to 60.0; |
| Amino acids | from 0.5 to 31.5; |
| Carbohydrates | from 16.0 to 70.0; |
| Lipids, nucleotides and other organic compounds | from 0.5 to 17.0. |

The present BAA preferably contains carbohydrates, in a composition of organic compounds, at the following ratio in % by mass within:

| | |
|---|---|
| High-molecular compounds having a molecular mass of > 10.0 kDa | up to 3.0 |
| Low-molecular compounds having a molecular mass of 0.8-10.0 kDa | from 11.5 to 57.9 |
| Free monomers having a molecular mass of < 0.8 kDa | from 3.5 to 17.0. |

The present BAA preferably has a ratio between the mass content of carbohydrates and that of peptides, within said organic compounds having a molecular mass of 0.8-10.0 kDa, which ranges from 0.6 to 2.0.

The present BAA preferably contains organic compounds at the following ratio in % by mass:

| | |
|---|---|
| Peptides | from 40.0 to 60.0 |
| Amino acids Carbohydrates | from 12.9 to 17.1 from 16.0 to 32.8 |
| Lipids, nucleotides and other organic compounds | from 4.4 to 10.6. |

The present BAA preferably contains organic compounds at the following ratio in % by mass:

| | |
|---|---|
| Peptides | from 34.0 to 50.1 |
| Amino acids | from 16.6 to 22.6 |
| Carbohydrates | from 22.7 to 34.2 |
| Lipids, nucleotides and other organic compounds | from 5.4 to 10.6. |

The present BAA preferably contains organic compounds at the following ratio in % by mass:

| | |
|---|---|
| Peptides | from 28.0 to 33.6 |
| Amino acids | from 16.5 to 31.5 |
| Carbohydrates | from 6.9 to 37.9 |
| Lipids, nucleotides and other organic compounds | from 8.6 to 17.0. |

The present BAA preferably contains organic compounds at the following ratio in % by mass:

| Peptides | from 29.0 to 36.3 |
| Amino acids | less than 0.5 |
| Carbohydrates | from 57.7 to 70.0 |
| Lipids, nucleotides and other organic compounds | from 0.5 to 5.5. |

The problem set up in this invention is also solved by a method for producing a biologically active agent having immunomodulating properties according to to the present invention, wherein the method comprises disintegrating and homogenizing raw material of animal tissue except tissue of human embryos, purifying homogenate from non-disintegrated tissue elements by means of filtration and/or centrifugation at 150 to 250 g for 10 to 15 min, isolating the sediment comprising cell membranes from said homogenate, hydrolyzing said sediment, filtering and/or centrifugating the hydrolysis products at above 1.500 g for 20 to 40 min, and collecting the supernatant of the hydrolysis products comprising cell membrane components as desired product.

The following raw materials can be used: embryonic tissue, placental tissue, differentiating thymocytes of the thymus, spermatogones and spermatocytes of testes, epithelial tissue of the intestine, bone marrow cells, regenerating cutaneous tissue, regenerating hepatic tissue, tissue of regenerating amphibian limbs, pathologically transformed tissue taken prior to the stage of regeneration, or any combination thereof.

The sediment comprising cell membranes is isolated from the purified homogenate by means of centrifugation at 1,500 to 90,000 g and/or filtration through a filter with a characteristic pore size less than 1 $\mu$m with subsequent washing by means of resuspending and repeatedly centrifugating and/or filtering up to obtaining the sediment comprising cell membranes in an amount of no less than 60 % by mass in relation to organic compounds present in said sediment.

Besides, the sediment comprising cell membranes is subjected, prior to hydrolysis, to additional centrifugation at 20,000 to 100,000 g in density gradient for 1 to 2 hours with isolating plasmatic cell membranes.

The hydrolysis is carried out at a temperature of T = 4-45°C.

This process can be carried out in the presence of a preservative.

Preferably, the present process comprises the thermal treatment of the supernatant of the hydrolysis products comprising cell membrane components is subjected to thermal treatment at a temperature ranging from 60°C to 120°C till complete denaturation of thermolabile compounds, followed by additional filtration and/or centrifugation at more than 1.500 g for 20 to 40 min with collecting supernatant comprising thermostable components of cell membranes as desired product.

Preferably, the hydrolysis products comprising cell membrane components are subjected, prior to filtration and/or centrifugation, to thermal treatment at a temperature ranging from 60°C to 120°C till complete denaturation of thermolabile compounds.

Besides, the supernatant of the hydrolysis products or the hydrolysis products comprising cell membrane components are preferably subjected to fractionating by means of dialysis through a semipermeable membrane and/or ultrafiltration and/or gelfiltration with collecting a fraction comprising low-molecular components having a molecular mass of ≤ 10.0 kDa as desired product.

Said low-molecular fraction of the supernatant or of the hydrolysis products comprising components of cell membranes is preferably subjected to affinity chromatography on lectines with collecting a fraction comprising carbohydrate-containing components as desired product.

A further subject matter of the present invention is a preparation for the normalization of the physiological state of human and animal organisms, which contains a biologically active agent having immunomodulating properties according to the present invention in an amount of 0.001 to 85 % by mass with the rest being an excipient.

Preferably, said biologically active agent comprises organic compounds at the following ratio in % by mass:

| Peptides | from 28.0 to 60.0 |
| Amino acids | from 0.5 to 31.5 |
| Carbohydrates | from 16.0 to 70.0 |
| Lipids, nucleotides and other organic compounds | from 0.5 to 17.0. |

More preferably, said BAA comprises organic compounds at the following ratio in % by mass:

| Peptides | from 40.0 to 60.0 |
| Amino acids | from 12.9 to 17.1 |
| Carbohydrates | from 16.0 to 32.8 |
| Lipids, nucleotides and other organic compounds | from 4.4 to 10.6. |

Further preferably said BAA comprises organic compounds at the following ratio in % by mass:

| Peptides | from 34.0 to 50.1 |
| Amino acids | from 16.6 to 22.6 |
| Carbohydrates | from 22.7 to 34.2 |
| Lipids, nucleotides and other organic compounds | from 5.4 to 10.6. |

Further preferably said BAA comprises organic compounds at the following ratio in % by mass:

| Peptides | from 26.0 to 33.6 |
| Amino acids | from 16.5 to 31.5 |
| Carbohydrates | from 20.9 to 37.9 |
| Lipids, nucleotides and other organic compounds | from 8.6 to 17.0. |

Still further preferably said BAA comprises organic compounds at the following ratio in % by mass:

| Peptides | from 29.0 to 36.3 |
| Amino acids | less than 0.5 |
| Carbohydrates | from 57.7 to 70.0 |
| Lipids, nucleotides and other organic compounds | from 0.5 to 5.5. |

As an excipient, the preparation may comprise a physiological solution of sodium chloride and/or a saline buffer solution and/or carbohydrate compounds and/or fats of minerals, animals, vegetables or synthetic origin or any combination thereof.

The preparation may also comprise a preservative.

A further subject-matter of the present invention is the use of a biologically, active agent having immunomodulating properties according to the present invention, for the manufacture of a preparation for the normalization of the physiological state of human and animal organisms, said preparation being suitable for administration to said organisms and consisting of said biologically active agent in an amount ranging from 0.001 to 85 % by mass with the rest being an excipient.

Preferably said preparation is administered as intramuscular injections at a dose ranging from 0.005 mg to 0.5 mg/kg of the body weight with 1-7 day intervals between injections and/or inhalations at a dose ranging from 0.012 mg to 0.12 mg/kg of the body weight with 4-48 hour intervals between inhalations and/or sublingual administrations at a dose ranging from 0.012 mg to 0.12 mg/kg of the body weight with 4-48 hour intervals between administrations and/or wet feedings at a dose ranging from 0.02 mg to 1.0 mg/kg of the body weight with 1-10 day intervals between feedings and/or oral pills at a dose ranging from 0.02 mg to 1.0 mg/kg of the body weight with 1 to 10 day intervals between administrations and/or intranasal administrations at a daily dose ranging from 0.001 mg to 0.05 mg/kg of the body weight with 2-24 hour intervals between administrations till the normalization of physiological parameters.

Further preferably said preparation is administered as applications to mucous or wound surfaces in the form of compresses, suppositories, ointments, powders and gels with 1-10 day intervals between applications till the normalization of physiological parameters.

At the same time, the normalization of the physiological state of human and animals can be realized by means of stimulation of the immune system, mainly by activation of the reticulo-endothelial system, T- and B-systems, neutrophiles and/or natural killers.

It has been established, that practical efficiency of the present BAA, produced according to the present method of production lies in its immunomodulating activity.

The comparative analysis of properties of the known BAA and the present BAA shows that the latter possesses a broader spectrum of action, higher effectiveness, safety in respect of overdose, and is free from side effects.

It has been established experimentally that practical efficiency of the present BAA produced by the present method lies in its high biological activity and, first of all, in increased immunomodulating and regenerating effects. Being injected into the organism the present BAA ensures:

a) higher stimulation of the processes of regeneration, including restoration of functional activity of hepatic tissue in cases of hepatitis, and liver regeneration following hepatectomy, healing of ulcers, wounds, injures, and skin in cases of wounds and damages, the normalization of blood formula and in other cases;

b) high efficacy in the treatment of acute respiratory diseases as well as in cases of inflammatory processes (pneumonia, mastitis, endometritis etc.);
c) beneficial effect in infectious diseases of humans and animals;
d) increased resistence of animals to infectious diseases and as a consequence, a decrease in cattle mortality;
e) improved general biological state of animals, which influences favourably on their productivity;

The practical use of the present BAA confirmed its lack of toxicity and the absence of side effects (allergies, delayed hypersensitivity and so on).

The mentioned results are achieved owing to special properties of the present BAA, which are explained by the presence of components extracted from cell membranes with modified antigenic structure in its composition. It should be noted, that changes in physiological status of tissue cells find their reflection in a modification of the antigenic structure of cell membranes, that is accompanied by a modification of cell membrane components as well as by changes in immunogenicity of the tissue. The latter induces a response in the immune system of an organism.

The degree of the modification of antigenic structure of cell membranes and, consequently, the pronounced response of the immune system depend on the degree of changes in the physiological status of cellular tissue.

It was established that an increase in immunogenicity of the modified antigenic structure of cell membranes, firstly plasmatic cell membranes, is ensured in animal tissues at the stage of passing the processes of embryogenesis and/or proliferation, and/or differentiation of the cells, and/or pathology, preceding and/or accompanying the process of regeneration and/or reparation of the tissue.

There are the following tissues of such a type:

- differentiating thymocytes of the thymus (the process of differentiation of cells);
- spermatogones and spermatocytes of the testes, cells of bone marrow, epithelial tissue of the intestine (the processes of proliferation and differentiation of the cells);
- embryonal tissue, placental tissue (the processes of proliferation, differentiation, embryogenesis);
- tissues of regenerating amphibian limbs, rat regenerating liver after a partial hepatectomy or after a damage by $CCl_4$, regenerating cutaneous tissue, the pathologically altered tissue, for instance, in the case of gangrene, in cases of chemical or thermal injures (any combinations of the above mentioned processes).

Using the listed animal tissues except tissue of human embryos as raw material in the present method of BAA preparation, ensures the production of a BA.A-with expected properties.

It was also established that a characteristic feature of the above listed tissues is an altered immunogenic structure of their cell membranes and the modification of components of cell membranes. This feature allows their differentiation from intact or stable cellular tissues of mature animals. This property of modified components of cell membranes affords their usage in the present BAA which, being administered to humans or animals, activates their immune system toward searching and eliminating pathology.

Physical carriers of information in the above listed processes and/or deviations from the norm in pointed tissues are specific components of cell membranes. The present BAA comprises modified components of cell membranes with a modified antigenic structure, which activate directly the immune system.

A response of the organism is augmented considerably following an injection of the present BAA which comprises components of cell membranes in the form of thermostable components stable at a temperature of T<120°C; in the form of low-molecular components, namely, with a molecular mass of M = 0.8-10.0 kDa, as well as in the form of carbohydrate-containing components of cell membranes, mainly in the form of glycopeptides, glycolipids, oligocarbohydrates, nucleotides and/or free monocarbohydrates.

Due to a high static and dynamic concentration of components of cell membranes in the present BAA, its content of immunoinhibitors and ballast compounds is lowered. Thus, being highly specific, the present BAA is practically devoid of side effects.

The present BAA is used as an active ingredient in a preparation, where, various excipients depending upon their prescription are present.

At the same time, an unobvious dependence between effectiveness of the preparation and its BAA content is revealed. It allows to considerably reduce the content of active ingredient, while keeping the necessary efficiency of the preparation, and preventing the possibility of overdosages, side effects.

Besides, the above described properties of the preparation afford its use for the normalization of the physiological state of the organism at any doses, namely from 0.001 to some grams per 1 kg of the body weight.

The indicated properties are illustrated below in Examples of concrete realization of the invention, particularly in Examples 9, 11, 17, 24. 27-33 and others.

In the above described method of using the novel preparation, the treatment of diseases mainly induced by functional insufficiency of organs and tissues is achieved owing to concrete schemes and ways of its administration.

This is illustrated below in Examples of concrete realization of the invention, particularly in Examples 13. 16-18. 20. 21. 23. 24. 31. 32 and others.

For internal usage the preparation is administered in the form of injections with a physiological solution of sodium chloride or buffer salt solution as an excipient. For external usage, besides above mentioned solutions as a excipient can be used components of gels, i.e.- carbohydrate compounds of animal as well as plant origin, for example honey, juices, pulp of plants, etc. Fats of minerals, animals, vegetables or synthetic origin or their mixtures can be used as excipients for ointments.

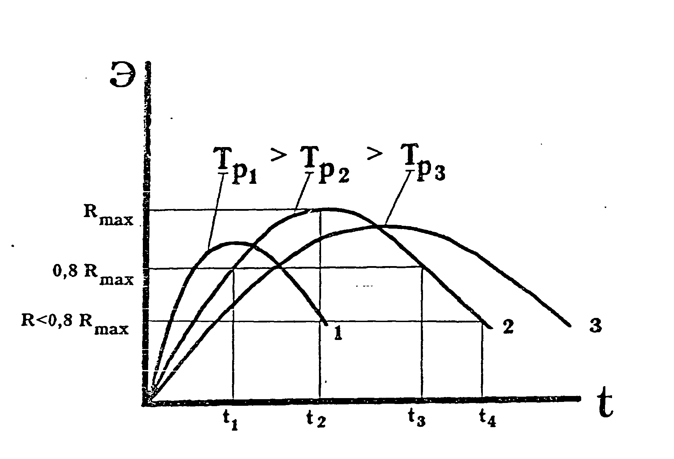

Diagrammatic curves of temperature-time dependency of the present BAA efficiency during hydrolysis of the sediment containing cell membranes are shown in the drawing.

According to the invention, a biologically active agent with immunomodulating properties, for the normalization of the physiological state of the organism, is produced by the following way.

The animal tissue modified in the processes of embryogenesis, and/or proliferation and/or differentiation of the cells, and/or pathology preceding and/or accompanying the process of regeneration and/or reparation of the tissue was taken as raw material.

The animal tissue taken is washed, disintegrated, and homogenized with physiological and/or buffer solutions for complete cell destruction. The homogenized mixture is purified from non-disintegrated tissue elements by the method of filtration and/or centrifugation at the acceleration of 150 to 250 g for 10 to 15 min. By centrifugation at acceleration of 1,500 to 90,000 g and/or filtration through a filter with the characteristic pore size less than $1\mu m$, the sediment comprising cell membranes, is isolated from purified homogenate, with subsequent sediment hydrolysis. Then the hydrolysis products are filtrated and/or centrifugated at above 1.500 g for 20 to 40 min. with collecting supernatant of the hydrolysis products, comprising components of cell membranes as desired product for the normalization of the physiological state of the organism by external usage (inhalations, applications, intranasal drops, ointments, suppositories, etc.).

The efficiency of the desired product depends on its content of modified components of cell membranes. Their content can be elevated by choosing raw material with an increased intensiveness of modification processes and optimal conditions of realization of the production method.

The choice of animal tissue as raw material depends on the intensiveness of modification processes of antigen structure of cell membranes, which is accompanied by modification of cell membrane components with a change in immunogenicity of the tissue.

It is established that an increase in immunogenicity of the modified antigen structure of cell membranes, and, firstly, plasmatic cell membranes, in animal tissues occurs in the processes of embryogenesis and/or proliferation and/or differentiation of the cells, and/or pathology, preceding and/or accompanying the process of regeneration and/or reparation of the tissue.

The following animal tissue can be used as raw material:

- differentiating thymocytes of the thymus;
- spermatogones and spermatocytes of testes, cells of bone marrow, intestinal epithelial tissue (the processes of proliferation and differentiation of cells) :
- embryonal tissue, placental tissue (the processes of proliferation, differentiation, embryogenesis);
- tissues of regenerating amphibian limbs, rat regenerating liver after the partial hepatectomy or after a damage induced by $CCl_4$, regenerating cutaneous tissue, pathologically altered tissue, for instance in the case of gangrene, of chemical or thermal injures (any combinations of the above mentioned processes).

The use of described animal tissues except tissue of human embryos as raw material in the present method of BAA preparation ensures obtaining a biologically active agent with the expected properties.

It was established that a characteristic feature of the above named variety of tissues is their changed antigen structure of cell membranes, accompanied by the modification of the organism by external usage (inhalations applications, intranasal drops, ointments, suppositories, etc.).

The efficiency of the desired product depends on its content of modified components of cell membranes. Their content can be elevated by choosing raw material with an increased intensiveness of modification processes and optimal conditions of realization of the production method.

The choice of animal tissue as raw material depends on the intensiveness of modification processes of antigen structure of cell membranes, which is accompanied by modification of cell membrane components with a change in immunogenicity of the tissue.

It is established that an increase in immunogenicity of the modified antigen structure of cell membranes, and, firstly, plasmatic cell membranes, in animal tissues occurs in the processes of embryogenesis and/or proliferation and/or differentiation of the cells, and/or pathology, preceding and/or accompanying the process of regeneration and/or reparation of the tissue.

The following animal tissue can be used as raw material:

- differentiating thymocytes of the thymus;
- spermatogones and spermatocytes of testes, cells of bone marrow, intestinal epithelial tissue (the processes of proliferation and differentiation of cells);
- embryonal tissue, placental tissue (the processes of proliferation, differentiation, embryogenesis);
- tissues of regenerating amphibian limbs, rat regenerating liver after the partial hepatectomy or after a damage induced by $CCl_4$, regenerating cutaneous tissue, pathologically altered tissue, for instance in the case of gangrene, of chemical or thermal injures (any combinations of the above mentioned processes).

The use of described animal tissues as raw material in the present method of BAA preparation ensures obtaining a biologically active agent with the expected properties.

It was established that a characteristic feature of the above named variety of tissues is their changed antigen structure of cell membranes, accompanied by the modification of cell membranes. This affords their differentiation of intact or stable cellular tissue of mature specimens. This feature of the indicated tissues allows their use for preparing the present BA A which, at human or animal administration activates their immune system toward searching and eliminating pathology.

It is preferably to use the animal tissue taken at the incremental stage of the modification process. This stage can be defined empirically by a trend in changing immunogenicity. For example, in the case of embryonal tissue use, this stage constitutes the first half of embryogenesis. Rat regenerating liver is used 4-12 hours after partial hepatectomy. In the use of regenerating amphibian limbs, this corresponds to the blastema formation.

For preparing the present agent each of the indicated tissues following preliminary disintegration is homogenized in three volumes of buffer physiological solution until complete destruction of cells and homogeneous mass will be obtained. The homogenate is purified from non-disintegrated tissue elements and whole cells by the method of filtration and/or centrifugation at 150 to 250 g for 10 to 15 minutes. Sediment isolated from the purified homogenate comprising cell membranes, is subjected to hydrolysis.

The purification of homogenate from non-disintegrated tissue elements is performed by means of centrifugation at 150 to 250 g for 10 to 15 min. It is explained by the fact that undesirable precipitation of cell membranes takes place at higher accelerations and/or longer durations of centrifugation, which purification is less effective at acceleration less than 150 g.

The purification of homogenate from the non-destroyed elements could be ensured by the method of filtration on a large-pored filter, for instance through several layers of gauze, cotton, coarse calico or nylon.

The sediment comprising cell membranes is isolated from purified homogenate by filtration and/or centrifugation at 1,500 to 90,000 g for 20 to 60 min.

The choice of such a range of accelerations is determined by the isolation conditions of defined fraction of cell membranes, comprising the maximum quantity of plasmatic cell membranes.

At acceleration less than 1.500 g, isolation of membranes is not effective, while in the case of acceleration above 90,000 g other ballast cell formations, worsening BAS quality are sedimented.

To increase effectiveness of the desired product, the sediment of cell membranes is purified from tissue extract components sorbed and included inside vesicules and organells. For this purpose the sediment is washed by suspending with fresh portions of buffer solution or distillated water followed by repeated centrifugation and/or filtration until the sediment. Which Comprises Cell membranes in relation to organic compounds present in an amount of no less than 60 per cent by mass, is obtained.

The relative content of cell membranes in the sediment, isolated for hydrolyzing, is defined through contents of carbohydrates and proteins in both the sediment and the supernatant produced in the processes of resuspending and centrifugation at 100,000 to 150,000 g for 40 to 60 min.

The efficiency of desired product increases considerably with rising the amount of components of plasmatic cell membranes in its content. For this purpose, the sediment comprising cell membranes, prior to hydrolysis, is additionally resuspended and centrifugated at 20,000 to 100,000 g for 1 to 2 hours in a gradient of density, for example, that of saccharose, ficol, percol, and some other similar compounds with subsequent isolation of plasmatic cell membranes.

When the gradient of saccharose density is used, a fraction of plasmatic cell membranes is collected in the range of density of d=1.14 to 1.18 $g/cm^3$ in accordance with the known methods (H. M. Berman, W. Gram, M.A.Spirtes.- Biochemica et biophysica acta,- 1969, -v. 183, -1, -p. 10-18; J. Gurd, W. Evans, H.Perkins.- Biochemistry Journal, -1973,- v. 135, -4. -p. 827-832; G. Schapira, J.Dobocz.- Biochemica et biophysica acta,-1974, -v. 345, -3, -p. 348-358).

This fraction of plasmatic cell membranes is washed from saccharose and sorbed components of the extract by the method of centrifugation with physiological solution at 20,000 to 100.000 g for 20 to 40 min., and the sediment comprising plasmatic cell membranes is isolated.

The isolated sediment, comprising cell and/or plasmatic cell membranes, after resuspending with physiological and/or buffer solution, is hydrolyzed. The hydrolysis products are filtrated and/or centrifugated at above 1.500 g with collecting supernatant of the hydrolysis products, comprising components of cell membranes, as desired product.

The efficiency of the desired product rises even in the case of use of cell membrane components in the form of low-molecular components, so far as it becomes devoid of side effects and requires a wider range of action without being species-specific.

In order to do so, cell membranes are subjected to hydrolysis, with decomposing high-molecular structural formations of cell membranes into the products comprising components with lower molecular mass. The efficiency of the hydrolysis products will depend on the specificity of hydrolysis, which in its turn depends largely on the temperature - time characteristics.

It was established that the immunomodulating properties of the present agent particularly its capability to increase the activity of macrophages in rat peritoneal exudate in vitro, were maximal in the case of hydrolysis at a temperature ranging from 4°C to 45°C.

Any deviations from this range are undesired, because:

- at a temperature of less than 4°C the duration of the hydrolysis increases which reduced the economic efficiency;
- at a temperature of above 45°C the activity of the enzymes ensuring the hydrolysis of raw material lowers, while the decomposition processes of active components accelerate and, as a consequence, the quality of desired product gets worse.

The duration of hydrolysis is defined by the maximum level of immunomodulating effect of the present BAA by activation of macrophages of peritoneal exudate according to the NBT test in particular.

A prolonged hydrolysis has to be performed in the presence of a preservative.

An increase in relative content (relative concentration) of low-molecular components in the hydrolysis products comparing to that of thermolabile compounds is ensured by thermal treatment of the hydrolyzate until complete denaturation of thermolabile compounds. Afterwards, these should be removed in the form of sediment by the method of filtration and/or centrifugation at no less than 1,500 g for 20 to 40 min. Supernatant comprising thermostable components is used as desired product.

The thermal treatment of the hydrolysis products is performed at a temperature ranging from 60°C to 120°C. Outside this range, BAA specific activity declines.

Thus, at a temperature less than 60°C, not all proteins can be denatured, resulting in an increase of the content of thermolabile compounds of desired product. As a consequence, BAA specific activity declines.

At a temperature above 120°C, a jump like increase in the rate of structural changes in peptide and carbohydrate components of desired product takes place, which also declines its quality.

After the thermal treatment of the hydrolysis product, thermostable components are separated by the method of centrifugation at a speed of not less than 1,500 g for 20 to 40 min so far as the degree of removing the denatured thermolabile compounds at lower accelerations falls down considerably.

The coagulated denatured thermolabile compounds are eliminated from supernatant by the method of filtration through a paper filter or cloth filter.

The desired product comprising thermostable components of cell membranes has a higher specific activity in comparison with supernatant of the hydrolysis products and allows the solution of a wider spectrum of problems. It can be used as injections in veterinary and medicine.

The following increase in specific activity of a BAA is achieved by means of collecting low-molecular components with a molecular mass ≤ 10.0 kDa from the hydrolysis products by dialysis through a semipermeable membrane and/or ultrafiltration, and/or gelfiltration. This allows high-molecular compounds capable of inducing side effects to remove completely.

As noted earlier, the efficiency of the present BAA is determined in considerably degree according to the content of carbohydrate-containing components of cell membranes with a molecular mass of 0.8-10.0 kDa. In this connection a low-molecular fraction is subjected to the affinity chromatography on lectines with collecting carbohydrate-containing components in the form of glycopeptides, glycolipides, oligocarbohydrates, nucleotides and free monocarbohydrates, which are used as desired product.

Thus, the desired product is devoid of side effects and possesses high efficiency, which allows its wide use in clinical practice.

The present BAA is used as an ingredient in producing a pharmacological preparation, where its concentration depends on the way of its administration. When wet or soft feedings are used BAA concentration in the preparation is low and increases when a preparation is used as ointments, gels or powders.

Depending on the dosage form of the preparation, various excipients are used. Thus, a physiological solution of sodium chloride and saline buffer solution are used in injections, applications, intranasal drops. Carbohydrate compounds are used as an excipient dosage form designed for feeding of bees (for instance, 10 % sugar syrup) and gels (for example, honey, juices, and pulp of plants). For ointments, fats of minerals, animals, vegetables or synthetic origin and their mixtures are used as excipient.

Examples of concrete realization of the present group of inventions, as well as the methods used for determination of BAA compound and properties, are described below.

Example 1

For the production of the present biologically active agent, embryonic tissue of cattle taken in the first half of pregnancy was used as the raw material. After disinfection with a weak solution of potassium permanganate ($KMn_7O_4$), cutaneous and muscular tissue, liver, spleen, pancreas, kidneys, lungs and heart were cut off from embryons with subsequent disintegration. Then 10 kg of disintegrated mass was mixed with 30 kg of centimolar phosphate saline buffer solution (0.01 M PBS) pH 7.2. comprising centimolar dibasic sodium phosphate and 0.15 M solution of sodium chloride (0.01 M $Na_2HPO_4$ and 0.15 M NaCl) with the following homogenization by a colloidal mill until homogeneous mass was obtained. Non - disintegrated tissue elements were removed as sediment after centrifugation at 150 g for 15 min. After removing the floated fat by the method of filtration through cotton (coarse calico), homogenate supernatant was centrifugated at 1.500 g for 60 min.

The produced sediment comprising cell membranes in an amount of 60% in relation to the present organic compounds was resuspended in 15 kg of centimolar phosphate saline buffer solution (0.01 M PBS), with subsequent centrifugation at 5,000 g for 40 min. Then the sediment was resuspended again in 7.5 kg of distilled water and centrifugated at 20,000 g for 30 min. Following this, the sediment was resuspended once more in distilled water and centrifugated under the same conditions for 40 min. The fourth fold resuspending of the sediment was carried out in 7.5 kg of centimolar phosphate saline buffer solution (0.01 M PBS) with subsequent centrifugation at 20,000 g for 20 min.

The washed sediment comprising 90% of cell membranes, was suspended in 5.0 kg of centimolar phosphate saline buffer solution (0.01 M PBS). Then, after adding a preservative (chinozol) to the final concentration of 0.08 per cent by mass the sediment was hydrolyzed at a temperature of 4°C for 6 weeks.

The hydrolyzate produced was centrifugated at 1,500 g for 40 min. with collecting supernatant of the hydrolysis products comprising components of cell membranes as desired product. In Tables 1 and 2 a composition of organic compounds in a BAS and its specific activity are shown.

The desired product comprising a BAS in an amount of 1.0 per cent by mass, with the rest being an excipient, was used as intranasal pharmacological preparation in the treatment of female mastitis. The preparation was administered into each nostril in an amount of 2 drops with following nasal massage. Recovery was observed in 80% patients (n=25) on the 1st-3rd day

Table 1

| Immunomodulating and regenerating effects of biologically active agents (BAAs) | | Quantitative indices of $BA_A$ effects in examples | | | | | | |
|---|---|---|---|---|---|---|---|---|
| | | No. 1 | No. 2 | No. 3 | No. 5 | No. 9 | No.11 | No. 13 |
| 1. An increase in the activity of macrophages of rat peritoneal exudate versus control, % | Enzyme activity (NBT-test) | 69, 7 | 79, 1 | 61, 3 | 101, 9 | 109, 6 | 86, 8 | 117, 8 |
| | Phagocyte activity according to E. Coli | 60, 2 | 73, 5 | 49, 3 | 90, 4 | 98, 7 | 77, 7 | 106, 5 |
| 2. An increase in the activity of blood neutrophyles versus control, % | Enzyme activity (NBT-test) | 82, 7 | 93, 7 | 74, 9 | 115, 9 | 124, 5 | 102, 1 | 131, 3 |
| | Phagocyte activity by E. Coli | 60, 5 | 70, 1 | 52, 1 | 78, 4 | 81, 3 | 67, 5 | 85, 9 |
| 3. Stimulation of blasttransformation reaction versus control , % | T-lymphocytes | 57, 1 | 64, 8 | 52, 4 | 83, 5 | 87, 4 | 65, 8 | 10, 6 |
| | B-lymphocytes | 75, 4 | 85, 9 | 69, 3 | 118, 8 | 126, 1 | 100, 4 | 137, 2 |
| 4. An increase in titre of thymic serum-like factor(TSF) versus control, % | | 3026 | 3874 | 2345 | 497, 2 | 432, 7 | 426, 3 | 566, 5 |
| 5. Stimulation of natural killer activity of rat splenocytes versus control, % | | 69, 3 | 81, 3 | 61, 9 | 99, 2 | 106, 4 | 89, 2 | 113, 6 |
| 6. An increase in reparative abilities of rat hepatocytes by reconstruction of activity after $CCl_4$ injection versus control, % | Na, K-ATPases | 34, 2 | 40, 5 | 30, 3 | 57, 4 | 60, 8 | 49, 2 | 63, 4 |
| | Blood aminotranspherases | 70, 7 | 81, 9 | 61, 3 | 92, 4 | 93, 5 | 86, 8 | 96, 0 |
| | $K^+$ Concentration of hepatocytes | 24, 6 | 30, 1 | 20, 7 | 42, 6 | 44, 1 | 35, 8 | 46, 6 |

Continuation of Table 1

| Immunomodulating and regenerating effects of biologically active agents (BAAs) | | Quantitative indices of BAA effects in examples | | | | |
|---|---|---|---|---|---|---|
| | | No.16 | No.17 | No.18 | No..20 | No.21 |
| 1. An increase in the activity of macrophages of rat peritoneal exudate versus control,% | Enzyme activity (NBT-test) | 173.1 | 144.1 | 201.5 | 200.1 | 224.7 |
| | Phagocyte activity according to E.Coli | 154.2 | 121.8 | 185.4 | 171.7 | 200.9 |
| 2. An increase in the activity of blood neutrophyles versus control. % | Enzyme activity (NBT-test) | 149.1 | 129.0 | 168.4 | 161.0 | 181.6 |
| | Phagocyte activity by E.Coli | 104.2 | 85.8 | 120.6 | 113.6 | 129.2 |
| 3. Stimulation of blasttransformation reaction versus control .% | T-lympho cytes | 132.5 | 111.4 | 152.0 | 160.3 | 191.9 |
| | B-lympho cytes | 193.6 | 163.9 | 220.9 | 224.7 | 262.5 |
| 4. An increase in titre of thymic serum-like factor(TSF) versus control.% | | 766.2 | 649.7 | 858.9 | 889.8 | 1007.6 |
| 5. Stimulation of natural killer activity of rat splenocytes versus control.% | | 145.2 | 117.2 | 170.0 | 182.0 | 212.2 |
| 6. An increase in reparative abilities of rat hepatocytes by reconstruction of activity after $CCl_4$ injection versus control.% | Na,K-ATP-ases | 87.1 | 78.4 | 94.0 | 91.4 | 100.0 |
| | Blood aminotranspherases | 97.1 | 93.0 | 100.0 | 96.2 | 100.0 |
| | $K^+$ Concentration of hepatocytes | 67.2 | 58.0 | 75.4 | 75.1 | 93.5 |

Table 2

| Organic compounds | Content of organic compounds in BAA produced in the examples mass % | | | | | | |
|---|---|---|---|---|---|---|---|
| | No. 1 | No. 2 | No. 3 | No. 5 | No.9 | No.11 | No.13 |
| Polypeptides | 37.7 | 34.7 | 40.9 | 20.6 | 21.9 | 25.1 | 13.7 |
| Peptides | 18.0 | 18.5 | 19.1 | 20.1 | 23.9 | 25.0 | 20.3 |
| T O T A L : | 55.7 | 53.2 | 60.0 | 40.7 | 45.8 | 50.1 | 34.0 |
| Amino acids | 12.9 | 13.2 | 13.4 | 17.1 | 17.5 | 16.6 | 22.6 |
| Content of carbohydrates -high-molecular compounds with a molecular mass M > 10.0 kDa | 5.0 | 4.0 | 1.0 | 2.2 | 0.8 | 0.4 | 1.4 |
| -low-molecular compounds with a molecular mass M=0.8-10.0 kDa | 15.5 | 19.5 | 11.5 | 24.1 | 21.0 | 15.0 | 22.3 |
| -free monomers or compounds with a molecular mass M<0.8 kDa | 6.5 | 5.5 | 3.5 | 6.5 | 9.5 | 7.3 | 10.5 |
| I N  A L L : | 27.0 | 29.0 | 16.0 | 32.8 | 31.3 | 22.7 | 34.2 |
| Lipides, nucleotides and other organic compounds | 4.4 | 4.6 | 10.6 | 9.4 | 5.4 | 10.6 | 9.2 |
| T O T A L : | 1000 | 1000 | 1000 | 1000 | 1000 | 1000 | 1000 |
| Ratio between carbohydrate and peptide contents G | 0.86 | 1.05 | 0.60 | 1.20 | 0.88 | 0.60 | 1.10 |
| Calculated efficiency, K | 0.61 | 0.76 | 0.47 | 1.06 | 0.98 | 0.72 | 1.15 |

Continuation of Table 2

| Organic compounds | Content of organic compounds in BA$_A$ produced in the examples mass % | | | | |
|---|---|---|---|---|---|
| | No. 16 | No. 17 | No. 18 | No. 20 | No. 21 |
| Polypeptides | less than 0,3 | | | | |
| Peptides | 27,7 | 33,3 | 27,7 | 36,0 | 28,7 |
| T O T A L : | 28,0 | 33,6 | 28,0 | 36,3 | 29,0 |
| Amino acids | 31,5 | 24,0 | 16,5 | less than 0,5 | |
| Content of carbohydrates -high-molecular compounds with a molecular mass M>10.0 kDa | less than 0,2 | | | | |
| -low-molecular compounds with a molecular mass M=0.8-10.0kDa | 23,2 | 20,0 | 30,8 | 41,4 | 57,9 |
| -free monomeres or compounds with a molecular mass M<0.8 kDa | 8,5 | 6,7 | 6,9 | 16,1 | 11,9 |
| I N A L L : | 31,9 | 26,9 | 37,9 | 57,7 | 70,0 |
| Lipides, nucleotides and other organic compounds | 8,6 | 15,5 | 17,0 | 5,5 | 0,5 |
| T O T A L : | 100,0 | 100,0 | 100,0 | 100,0 | 100,0 |
| Ratio between carbohydrate and peptide contents G | 0,84 | 0,60 | 1,20 | 1,15 | 2,00 |
| Calculated efficiency, K | 1,60 | 1,47 | 1,90 | 1,90 | 2,20 |

G and K - the coefficients, characterising the relative ratio of carbohydrates in the compounds with a molecular mass M= 0.8-10.0 kDa
- in respect to peptides (G) and
- in respect to organic compounds of a BAA with various molecular mass. (K).
(in details, see the Part "Analysis of the composition and properties of the present BAA and the preparation made on its basis")

of the treatment, being assessed by elimination of purulent discharges and dense nodes in the mammary gland, and by total blood analysis.

Example 2

The embryonic tissue, obtained by the same method as in Example 1 was disintegrated and homogenized in three volumes of the physiological solution of sodium chloride using a homogenizer with knife-like blenders, until a homogenous mass of destroyed cells is obtained. To remove non-disintegrated elements of the tissue including elements of connective tissue the homogenized mass was centrifuged at 250 g for 10 min.

After centrifugation, the sediment was discharged and produced supernatant was filtered through cotton and then subjected to repeated centrifugation at 90.000 g for 20 min. with isolating the sediment containing cell membranes. This sediment was resuspended with a volume of physiological solution that was twice less than in the first case and then centrifugated under the same conditions.

Subsequently, the sediment was twice resuspended in distilled water and once in the physiological solution and was centrifugated at 90.000 g for 20 min. The sediment comprising cell membranes, produced in this way, was resuspended with 4 volumes of a physiological solution and hydrolyzed at a temperature of 20°C for 8 days after adding a preservative up to the final concentration of 0.08 per cent by mass.

The hydrolyzate produced was centrifuged at 15.000 g for 20 min. with collecting supernatant comprising components of cell membranes as desired product.

The desired product comprising a BAA in an amount of 1.0 per cent by mass, the rest being an excipient, was mixed with 5 volumes of a physiological solution of sodium chloride and was used as a pharmacological preparation in the form of compresses and applications in the treatment of lower extremity varicosities. Daily application was made for the night. The effect of healing controlled by state of vessels, swelling of the legs and painfull sensations was noted in 5-14 days.

Example 3

Homogenization of embryonic tissue Was performed as in Example 2. For removing non-disintegrated elements of the tissue, the homogenized mass was filtrated through cotton (coarse calico) using a nutch - filter in vacuum. Then, 50 kg of the filtrate were ultrafiltrated using ВПУ-100-ПА hollow fibres (Ty 6-12-151-87) of an AP-2.0 apparatus.

In the process of recirculation through hollow fibres, the extract of the tissue was removed from homogenate, whose components had a molecular mass less than 100 kDa, while high-molecular compounds were subjected to concentrating.

Homogenate in an amount of 50 kg was concentrated to obtain 5 kg of a suspension of cell membranes. Then, the suspension was mixed with 45 kg of physiological solution and concentrated again. By this way, a fraction of cell membranes was purified from components of the extract with a molecular mass less tha 100 kDa. That operation was repeated 4 times. The suspension produced comprised 60 per cent by mass of cell membranes among organic compounds present in it. After adding 10 kg of physiological solution, the suspension was autolysed at a temperature of 45°C for 48 hours.

The hydrolyzate produced was filtrated through a paper filter, and the filtrate comprising components of cell membranes was used as desired product. The hydrolyzate produced was centrifuged at 15.000 g for 20 min. with collecting supernatant comprising components of cell membranes as desired product.

The desired product including a BAA in an amount of 1.0 per cent by mass, with the rest being an excipient, was mixed with 9 volumes of cucumber pulp and used as a pharmacological preparation in the form of facial gel mask for cosmetic purposes. In the cases of long-term storage, a preservative has to be added into gel (nipagine and nipasole at a ratio of 5:1 respectively) up to a final concentration of 0.1 per cent by mass. Facial gel applications were made for 2-3 hours. The procedure was repeated 1-2 times per week. The beneficial effect controlled by skin state was noted after 2-6 procedures.

Example 4

The homogenate of embryonic tissue purified from non-disintegrated tissue elements as in Example 3, was filtered on GF/S hollow fibres, holding particles with a size above 1 $\mu$m. All operations were performed as in Example 3, and as a result, desired product with the same composition and properties was produced.

Example 5

The sediment comprising cell membranes, produced in Example 1. after 4-fold washing, was resuspended with a concentrated solution of saccharose with a final density of the suspension produced $d_1 > 1.25$, which was ultracentrifugated at 100.000g for 60 min. in discrete density gradient of saccharose $d_1 > 1.25$; $d_2 = 1.20$; $d_3 = 1.18$; $d_4 = 1.16$; $d_5 = 1.14$; $d_6 = 1.12$. A fraction of plasmatic membranes was isolated in the density gradient of saccharose d=1.18-1.14. Then, membranes were washed from saccharose by method of centrifugation with a physiological solution at 20,000 g for 60 minutes. The sediment produced thereby was resuspended with 4 volumes of a centimolar phosphate saline buffer solution (0.01 M PBS) pH 7.2. Then, after adding a preservative up to a final concentration of 0.08 per cent by mass, the suspension of plasmatic cell membranes was hydrolyzed at a temperature of 4°C for 6 weeks.

The hydrolyzate produced was centrifugated at 1,500 g for 40 min. with collecting supernatant of the hydrolysis products, comprising components of plasmatic cell membranes as desired product.The composition of organic compounds in the desired product and specific activity are shown in Tables 1 and 2.

The desired product comprising a BAA in an amount of 1.0 per cent by mass, with the rest being an excipient, was used in the treatment of bronchopneumonia as inhalations. The beneficial effect controlled by pulmonary state and blood analysis was displayed following 2 to 8 days of the treatment.

Example 6

The suspension of cell membranes in saccharose with a final density d>1.25, produced in Example 5, was centrifugated at 20.000 g for 2 hours in the discrete density gradient of saccharose. All subsequent operations were performed as in the Example 5. and resulted in a desired product of the same composition and properties.

Example 7

To measure the ratio between components of cell membranes and components of connective tissue and extract in the present BAA obtained as in Examples 1 to 6, the amino acid assay was performed. Prior to hydrolysis the amount of proteins and carbohydrates in the sediment and supernatant produced by the method of ultracentrifugating of the suspension of cell membranes at 100,000 to 150,000 g for 60 min was determined as in Examples 1 to 6.

The amount of proteins in the sediment and supernatant was measured after ultracentrifugation of the suspension. After removing the supernatant, 4.9 ml of physiological solution were added to 0,1 ml of the sediment and, after resuspending, 0.1 ml was taken for measuring proteins photometrically according to the SDS-Lowry method (U. K. Laemmli. - Nature, - 1970, -v. 227, -5259, -p. 680-685).

To measure proteins in the supernatant, 0,1 ml of the latter was taken. The BAA assay on proteins showed that a BAA as in Examples 1, 2 and 5 contained components of cell membranes in an amount of $88 \pm 8$ per cent by mass, while a BAA as in Example 3 contained $70 \pm 10$ per cent.

Components of connective tissue were detected by the presence of oxylysine and oxyproline, as judged from the amino acid assay. These amino acids were absent in composition of the present BAA.

The measurement of carbohydrates in BAA samples was carried out according to the Antron reaction (S.Seifter, S.Dayton. C.Hovic.- Arch. Biochemistry and Biophysics, -1950, -25, -1. -p.191-200), taking into account total carbohydrates following hydrolysis and free carbohydrates as monomers. By the difference of these values, content of carbohydrates was calculated in various organic compounds.

For general hydrolysis 1.0 ml of a BAA sample mixed with 1.0 ml of 4 N solution of hydrochloric acid (4.0 N HCl) was taken. The hydrolysis was carried out at a temperature of 105°C for 2 hours. The hydrolyzate obtained was evaporated in an exsiccator with sodium hydroxide (NaOH) in vacuum. The dry residue of hydrolyzate was diluted in 1.0 ml of distilled water and used for analysis.

Measurement of free carbohydrates in BAA following acid hydrolysis was performed as follows: 1 ml of a sample was mixed with 1 ml of distilled water. Then 4,0 ml of 0.2% Antron reagent with 95 per cent sulphuric acid ($H_2SO_4$) were added with constant stirring at a temperature of -5°C. The produced mixture was boiled at a temperature of 95°C for 10 min. After cooling, optical density was measured at a wave length of 020 nm. As a standard glucose at concentration of 20 $\mu$g/ml was used.

In the present BAA produced as in Examples 1, 2, 3 and 5, concentration of carbohydrates in the composition of its compounds with a molecular mass M>0.8 kDa was 20,5%; 24,5%; 12,5% and 26,4%, respectively.

Herein after in the text, the values obtained experimentally represent mean values calculated using Student's test.

Example 8

The hydrolyzate supernatant, obtained as in Example 1, was subjected to thermal treatment at various temperatures of heating $T_1 = 55°C$, $T_2 = 60°C$, $T_3 = 95°C$, $T_4 = 120°C$ and $T_5 = 135°C$. The thermal treatment was performed until complete denaturation of the thermolabile compounds in the composition of the hydrolysis product. Then, centrifugation was performed at above 1,500 g for 30 min. with collecting the supernatant, comprising thermostable components of cell membranes as desired product.

In each case the desired product possessed immunomodulating effect whose value is shown in Table 3 as specific activity (activation of macrophages) in dependence on the chosen range of heating temperatures of the hydrolyzate. The optimal temperature of boiling. T=95°C, ensures a maximum increase in enzyme and phagocyte activity of desired product up to 102 3±5.5% and 92.1±7.4%. respectively. At a temperature of

Table 3

| Temperature of heating (thermoprocess) °C | Activation of macrophages of rat perito neal exudate (in vitro) a result of influence of the Present BAA, obtained at various thermoprocesses versus control, % | |
|---|---|---|
| | Increase of enzyme activity NBT-test | Increase of phagocite activity by E.Coli |
| 55 | 76,8 ± 4,6 | 53,8 ± 5,1 |
| 60 | 82,5 ± 4,9 | 70,8 ± 6,5 |
| 95 | 102,3 ± 5,5 | 92,1 ± 7,4 |
| 120 | 88,4 ± 5,1 | 78,3 ± 7,0 |
| 135 | 68,4 ± 5,0 | 66,2 ± 6,1 |
| Differences are significant with control P<0,05 | | |

Table 4

| Number of fraction | Molecular mass of fractions M, kDa | Activation of macrophages of rat peritoneal exudate under influen ce of low-molecular fractions of present BAA versus control, % |
|---|---|---|
| 1 | $M_1 > 50,0$ | 58,4 ± 4,5 |
| 2 | $10,0 < M_2 < 50,0$ | 103,6 ± 9,2 |
| 3 | $M_3 \leq 10,0$ | 170,3 ± 18,1 |
| 4 | $M_4 \leq 5,0$ | 186,8 ± 14,2 |
| 5 | $M_5 \leq 1,0$ | 138,7 ± 12,3 |
| 6 | $M_6 \leq 0,8$ | 86,2 ± 7,7 |
| 7 | $M_7 \leq 0,5$ | 55,1 ± 6,4 |
| Differences are significant with control P<0.05 | | |

55°C, an increase in specific activity of macrophages was equal, on average to 76.8±4.6% (by NBT-test) and 53.8±5.1% (by phagocytosis). At a temperature above 120°C, a jump-like increase in rate of structural changes in peptides and carbohydrate-containing compounds, which resulted in structural changes in components of desired product was noted. This reduces BAA specific activity.

Thus, at a temperature of 135°C an increase in specific activity of a BAA is equal, on average, to 68.4±5.0% - by NBT -test and 66.2±6.1% - by phagocytosis. The maximum value of BAS specific activity is ensured at a temperature ranging from T=60-120°C. At that temperature, an increase in activity of macrophages for the peritoneal exudate of rats (in vitro) is equal, on average, to 102.3±5.5% - by an increase in enzyme activity (NBT-test), and 92,1±7,4% - by an increase in phagocyte activity (according to E.Coli).

Example 9

The hydrolyzate supernatant, obtained in Example 1, was subjected to thermal processing at a boiling temperature of $T_1$ =95°C The boiling was processed until complete denaturation of thermolabile compounds with the subsequent centrifugation there of at 1 500 g for 40 min after cooling. The sediment in the form of denaturated compounds was removed. The supernatant, comprising some thermostable components of cell membranes, was used as desired product.

The desired product, containing a BAA in the quantity of 1,0 per cent by mass, and with the rest being an excipient, was used as pharmacological preparation in the treatment of some dermatoses in the form of ointment, which was prepared by mixing lanoline, vaseline and the BAS desired product in proportion of mass parts 1:3:1 respectively. The ointment was rubbed into the skin everyday. The curative effect controlled by state of skin has been shown in 2-8 days.

Example 10

The hydrolyzate obtained in Example 1, was subjected to thermal processing at a boiling temperature $T_1$ =95°C. The boiling was processed until complete denaturation of thermolabile compounds with the subsequent centrifugation thereof at 1 500 g for 40 min after cooling. The sediment in the form of denaturated compounds was removed. The supernatant, comprising some thermostable components of cell membranes, was used as desired product with the content and properties such as in Example 9.

Example 11

The hydrolyzate supernatant, obtained in Example 3, was subjected to thermal processing at a boiling temperature of $T_1$-95°C. The boiling was processed until complete denaturation of thermolabile compounds with the subsequent filtration thereof through filtrating paper using a nutch - filter in vacuum after cooling. The filtrate, comprising some thermostable components of cell membranes, was used as desired product.
The content of organic compounds in the hydrolysis products and quantitative indices of BAA effects are shown in Tables 1 and 2.
The desired product, was dried by the method of lyophilization and, then, used in sublingual administration as curative preparation in female mastitis. The procedure was repeated everyday. The curative effect controlled by the state of the breast (the presence of infiltrations, suppurations) and also by blood analysis was noted in 1-3 days.

Example 12

The hydrolyzate, obtained in Example 3, was subjected to thermal processing ata boiling temperature of $T_1$=95°C. The boiling was processed until complete denaturation of thermolabile compounds with the subsequent filtration thereof through filtrating paper using a nutch - filter in vacuum after cooling. The filtrate, comprising some thermostable components of cell membranes, was used as desired product with the content and properties such as in Example 11.

Example 13

The hydrolyzate supernatant, obtained in Example 5. was subjected to thermal processing at a boiling temperature of $T_1$=95°C. The boiling was processed until complete denaturation of thermolabile compounds with the subsequent centrifugation thererof at 50 ooog for 20 minutes after colling. The sediment in the form of denaturated compounds was removed. The supernatant, comprising some thermostable components of cell membranes, was used as desired product.
The content of organic compounds in the hydrolysis products and quantitative indices of BAA effects are shown in Tables 1 and 2.
The desired product, containing a BAA in the quantity of 0,5 per cent by mass with the rest being an excipient, was used as pharmacological preparation in the treatment of cow mastitis by intramuscular injections at a dose of 0,05 ml/kg of the body weight every third day. The curative effect controlled by the state of udder and the quantity of purulent discharges in the nipples was noted after 1-4injections.

Example 14

The hydrolyzate, obtained in Example 5, was subjected to thermal processing at a boiling temperature of $T_1$ =95°C. The boiling was processed until complete denaturation of thermolabile compounds with the subsequent centrifugation thereof at 50 000 g for 20 min after cooling. The sediment in the form of denaturated compounds was removed. The supernatant, comprising some thermostable components of cell membranes, was used as desired product with the content and properties such as in Example 13.

Example 15

The hydrolyzate supernatant, obtained in Example 1, was fractionated using the ultracentrifuge "Amicon" with ultrafilters which allow passing the compounds with a molecular mass less or equal 50.0 kDa; 10.0 kDa, 5.0 kDa, 1.0 kDa, 0.8 kDa and 0.5 kDa.
The biological activity of obtained fractions, which consist of compounds with a molecular mass 1) M>50.0 kDa ; 2) 10.0 kDa <$M_2$ < 50.0 kDa ; 3) $M_3$ < 10.0 kDa; 4) $M_4$ < 5.0 kDa ; 5 ) $M_5$ < 1.0 kDa; 6) $M_6$ < 0.8 kDa and 7) $M_7$ <0.5 kDa - was analysed by their ability to activate macrophages of rat peritoneal exudate according to NBT-test. The results of researches are shown in Table 4.

The analysis of Table 4 allowed to make the following conclusions:

1) the distribution of specific activity according to fractions has its pronounced maximum, which lies in the range of a molecular mass 0.8-10.0 kDa;

2) the decrease of specific activity in the range of a molecular mass M>10.0 kDa is connected with an increase in inhibitor action of desired product and a decrease in the concentration Of the active agent;

3) the decrease of specific activity in the range of a molecular mass M<0.800 kDa is connected with the reduction of active agent concentration represented as oligomer combinations with a molecular mass M>0,800 kDa.

### Example 16

The hydrolyzate supernatant, obtained in Examples 1 and 9, was ultrafiltrated using hollow fibres ВПУ-15-ПА (ТУ 6-12-151-87) of an apparatus AP-2.0. The produced ultrafiltrate, including low-molecular components of cell membranes with a molecular mass less or equal M≤10,0 kDa was used as desired product.

The content of organic compounds in the hydrolysis products and quantitative indices of BAA effects are shown in Tables 1 and 2.

The desired product, containing a BAA in the quantity of 1.0 per cent by mass, and with the rest being an excipient, was used as pharmacological preparation in the treatment of rat experimental hepatitis with intramuscular injections at a dose of 0,05 ml/kg of the body weight every second day (the character and results of more detailed treatment are given in Examples 42 and 43).

### Example 17

The hydrolyzate, obtained in Example 3, was subjected to dialysis for 16-24 hours through cellophane membrane against a physiological solution with the following terms (0,8 g of the preservative per 1 kg of physiological solution of sodium chloride). The obtained dialysate was used as desired product. containing low-molecular components of cell membranes with a molecular mass M≤10.0 kDa.

The content of organic compounds in the hydrolysis products and quantitative indices of BAA effects are shown in Tables 1 and 2.

The desired product, containing a BAA in the quantity of 0,1 per cent by mass, and with the rest being an excipient, was used as pharmacological preparation for the normalization of calf homeostatic parameters by intramuscular injections, administered at a dose of 0,05 ml/kg of the body weight every 1-2 months with deviation from the norm. The curative pharmacological effect controlled by monthly animal weighing testifies about 60-100% arising of daily weight in comparison with untreated animal of control group.

### Example 18

The hydrolyzate supernatant, obtained in Examples 5 and 13. was ultrafiltrated using hollow fibres ВПУ-15-ПА (ТУ 6-12-151-87) of an apparatus AP-2.0. The produced ultrafiltrate. including low-molecular components of cell membranes with a molecular mass less or equal M≤10.0 kDa was used as desired product. It included low-molecular components of plasmatic cell membranes with a molecular mass M ≤ 10.0 kDa.

The content of organic compounds in the hydrolysis products and quantitative indices of BAA effects are shown in Tables 1 and 2.

The desired product, containing a BAA in the quantity of 0.1 per cent by mass, and with the rest being an excipient, was used as pharmacological preparation in the treatment of rat experimental gastric ulcer with 20 everyday intramuscular injections at a dose of 0.05 ml/kg and 0.5 ml/kg of the body weight (the character and results of the more detailed treatment are given in Example 44).

### Example 19

The hydrolyzate supernatant, obtained in Examples 1. was subjected to gel-ultrafiltration in a Sephadex G-25 on the column with a diameter d=26 and length h=600 mm, which was equilibrated by decimolar phosphate buffer pH 7.2. included fifteen-centimolar solution of sodium chloride (0.15 M NaCl) The fractionating of hydrolyzate supernatant according to molecular mass , was carried out by the method of elution of column with the same buffer solution. The chromatographic profile of an eluate was controlled spectrophotometrically at a wave length of 280 nm as condition.

The fraction of low-molecular components of hydrolyzate, comprising components of cell membranes, was eluted as desired product with the content and properties such as in Example 16.

Example 20

The fraction of low-molecular components, obtained in Example 16, was subjected to affinity chromatography on Con-A-Sepharoze (made by the "Pharmacia" company). The column with a diameter of d=2 cm and length h=10 cm, filled by Con-A-Sepharoze, was prepared for procession according to the recommendations of said company.

After washing with equilibrium buffer solution, comprising twentymillimolar tris-hydrochloric acid buffer (0,02 M Tris HCl) pH 7,4 and one-and-a-half-decimolar sodium chloride (0,15 M NaCl), Con-A-Sepharoze was treated by charging buffer solution, comprising decimolar sodium acetate buffer (0,1 M $CH_3COONa$) pH 6,5, millimolar calcium chloride (1 MM $CaCl_2$), millimolar magnesium chloride (1 MM $MgCl_2$) and monomolar sodium chloride (1 M NaCl). And then it was repeatedly washed with equilibrium buffer. After titrating up to pH 7,4, a fraction of the low-molecular components, obtained in Example 16, was put on column and washed with equilibrium buffer solution until all unsorbated compounds were removed. The extraction of carbohydrate-containing components, affinically connected with Con-A-Sepharose, was processed by decimolar sodium acetate buffer (0,1 M $CH_3COONa$) pH 3,5 in physiological solution of sodium chloride.

The spectrographical control of eluation was performed at a wave length of 280 nm. The affino-extracted fraction, with carbohydrate-containing components of cell membranes, after neutralizing its acidity with solution of sodium hydroxide (Na0H) up to pH 7,4 and adding preservative to final concentration of 0.08 per cent by mass, was obtained as desired product.

The content of organic compounds in the hydrolysis products and quantitative indices of BAA effects are shown in Tables 1 and 2.

The desired product, containing a BAA in the quantity of 0,1 per cent by mass, and with the rest being an excipient, was used as pharmacological preparation in the treatment of rat experimental ovary inflammation by intramuscular injections. The injections were administered at a dose of 0,05 ml/kg of the body weight every second day with deviation from the norm. The curative pharmacological effect controlled by the parameters of inflammatory reactions was noted following 6-10 injections.

Example 21

The fraction of low-molecular components, obtained in Example 18, was subjected to affinity chromatography on Con-A-Sepharoze (made by the "Pharmacia" company) as in Example 20.

The content of organic compounds in the hydrolysis products and quantitative indices of BAA effects are shown in Tables 1 and 2.

The desired product, containing a BAA in the quantity of 0,1 per cent by mass, and with the rest being an excipient, was used as pharmacological preparation in the treatment of foot "sugar" gangrene in diabetic patients by intramuscular injections. The injections were administered at a dose of 0,05 ml/kg of the body weight every third day. The curative pharmacological effect controlled by tissue and vessel state was noted after 5-15 injections.

Example 22

In order to define how the efficiency of the present BAA depends on the content of some modified components of cell membranes, the raw material from animal tissues was used. In these animal tissues the processes of embryogenesis and/or proliferation and/or differentiation of the cells, and/or pathology, preceding and/or accompanying the process of regeneration and/or reparation of the tissue had taken place. The obtained tissues were processed as in Example 9.

The property and effect of desired product produced in these processes, with above mentioned modification of cell stuctures, namely cell membranes are shown in Table 5.

The analysis of Table 5 shows, that the presence of immunomodulating effect and considerable increase of this effect in the present agent depends on the raw material used, from which the hydrolysis products including some modified components of cell membranes are obtained.

Thus, for this process the following raw material from animal tissue, taken precisely at the stage of cell proliferation and differentiation was used: spermatogones and spermatocytes of rat testes, cells of bone marrow, intenstinal epithelial tissue and rat embryonic tissue was used as raw material with the processes of the embryogenesis. As raw material with pathologic processes, preceding regeneration, the tissue of rat regenerating liver after partial hepatectomy, was used. And with pathology, following regeneration - the tissue of regenerating axolotl (Ambistoma) limbs, the tissue of rat regenerating liver after subcutaneous injections of carbon tetrachloride ($CCl_4$) were used.

For comparison simultaneously with obtained experimental data of Table 5, the results of immunomodulating effect obtained during usage of muscular and liver tissue of intact mature male rats as raw material are illustrated.

The analysis of the data in Table 5 shows, that immunomodulating effect of the present BAA using tissues, taken in the processes of embryogenesis, proliferation and pathology, preceding and/or accompanying the regeneration and/or reparation, is considerably higher, than in case of usage of other well known substances, because, in these well kown preparations a raw material from animal tissue, in which the above mentioned processes were absent.

Besides, one can conclude that the most significant immunomodulating effect was observed in regenerating tissues of the liver, axolotl (Ambistoma) limbs and embryonic tissue in comparison with the tissues of the above mentioned processes.

Example 23

For preparing the present agent rat regenerating liver

Table 5

| Variety of used animal tissues | Activation of macrophages of rat peritoneal exudate (in vitro) as a result of influence of the present BAA - TS, obtained from various tissues versus control, % | |
| --- | --- | --- |
| | Increase in enzyme activity (NBT-test) | Increase in phagocyte activity by E.Coli |
| Hepatocytes of the intact liver | $55,3 \pm 4.7$ | $43.9 \pm 4,1$ |
| Intact muscular tissue of the mature animals Spermatogonias and | $62,6 \pm 4,8$ | $55,7 \pm 4,3$ |
| spermatocytes of the testes | $81, 3 \pm 5,6$ | $70,2 \pm 4,8$ |
| Epithelial tissue of the intestine | $84,5 \pm 5,9$ | $73,2 \pm 5,4$ |
| Liver hepatocytes after $CCl_4$ injection | $88,4 \pm 5,4$ | $78,8 \pm 5,1$ |
| Cells of bone marrow | $90,3 \pm 6,8$ | $81,6 \pm 6,1$ |
| Tissue of regenerating limbs of axolotle* | 92,4 101,2 | 83,6 94,1 |
| Embryonal muscular tissue | $95,6 \pm 6,2$ | $88,2 \pm 5,7$ |
| Hepatocytes of rat regenerating liver after the hepatectomy | $112,4 \pm 6,8$ | $104.6 \pm 6,2$ |
| Differences are significant with control P<0.05 * The tissue for which all significances displayed in this Table are obtained in concrete experiment at the 4th-12th hour after hepatectomy made according to the well known method / G.M. Higgins. R.M. Anderson. - Experimental pathology of the liver. - Arch. Pathology. -1931, -N 12, -p. 186 / was used as raw material. | | |

The liver tissue was processed as in Examples 1, 9 and 16.

The specific activity of obtained desired products was defined experimentally by the activation of reparative abilities in rat liver hepatocytes after their damage induced by carbon tetrachloride ($CCl_4$). As a result of that damage the activity of Na, K-ATPase was reduced by:

$38,6 \pm 3,4\%$ in case of usage of the desired product obtained as in Example 1;
$58,6 \pm 4.2\%$ - as in Example 9;
$88,5 \pm 8,6\%$ - as in Example 16;

in comparison with control group of animals untreated with the declared substance.

The activity of blood aminotranspherases was regenerated correspondingly by:

71,6±10.3% as in Example 1;
89,2±6,2% (9) and 98,7±1,3% (16),

and potassium concentration of liver hepatocytes was regenerated correspondingly by:

28,4±4,4% (1); 36,8±5,4% (9) and 71,8±8,8% (16).

The desired product, including a BAA in the quantity of 0,1 per cent by mass, and with the rest being an excipient, was used as curative pharmacological preparation in the treatment of calf hepatitis by intramuscular injections. The injections were administered at a dose of 0,05 ml/kg of the body weight every 3-rd day with deviation from the norm. The curative pharmacological effect controlled by the indices of amino-transpherases, blood bilirubin and nitrogen concentration was shown after 4-8 injections.

Example 24

For preparing the present agent, oilcake of bovine testes was used. This oilcake is obtained after lydase extraction with 3% acetic acid from tissue homogenate.

The obtained testicular oilcake, being a waste of lydase production, was repeatedly homogenized with a triplicated volume of physiological solution in knives-like homogenizer and centrifugated at 250 g for 15 minutes in order to remove non-disintegrated elements of the tissue.

For obtaining the desired product the following operations were processed as in Examples 1. 9, 16.

The specific activity of obtained desired products was defined experimentally by the indices of macrophage activation in peritoneal exudate and neutrophiles of rat blood. Using a desired product, obtained as in Example 16, the enzyme activity macrophages was increased by 125,2±14,1%, phagocyte activity according to E. Coli - by 115,4110,8%. For blood neutrophiles these indices were increased by 115,4114,5% and 83,4±8,6% correspondingly.

The desired product, including a BAA in the quantity of 1,0 per cent by mass, and the rest being an excipient, mixed with 50 volumes of water was used as pharmacological curative preparation in the form of drinks for the normalization of parameters from hen homeostasis. The hens received the drink at a dose of 5 ml/kg of the body weight every 2-6 weeks with deviation of the pointed parameters from the norm.

The efficiency was controlled by the calculation of egg-laying, that testified of its rising by 20-40% comparing with control group of hens untreated with this preparation.

Example 25

For preparing the present agent, cow placental tissue, processed as in Examples 1. 9, 16, was used.

The specific activity of obtained desired products was defined experimentally by macrophage activation of peritoneal exudate and neutrophiles of rat blood, comparing with control group. During the use of the desired product, obtained as in Example 16 the enzyme activity of macrophages was increased by 183.6±17.4%. and phagocyte activity - by 131,4±11.8% according to E.Coli. For blood neutrophiles these indices were increased by 156,1±17,2.% and 111.2±12.3% correspondingly.

The desired product, including a BAA in the quantity of 0.5 per cent by mass, and the rest being an excipient, was used as the pharmacological preparation in the treatment of cow endometritis by administration of intramuscular injections. The preparation at a dose of 0.05 ml/kg of the body weight was injected every 3-rd day. The curative pharmacological effect controlled by the parameters of inflammatory reaction was shown after 4-8 injections.

Example 26

For preparing the present agent, a oilcake of the thymus was used. This oilcake was obtained after the extraction of some insoluble tissue components that were wastes of thymosine production from the homogenate.

The thymic oilcake was processed as in Examples 1. 9 and 16.

The specific activity of obtained desired products was defined experimentally by the stimulation of blasttransformation reaction in T-lymphocytes. In the case of usage of the desired product, obtained as in Example 16, it was increased by 212,4±15,5% comparing with control group.

The desired product, including a BAA in the quantity of 0,5 per cent by mass, and the rest being an excipient was used as curative pharmacological preparation in infectious diseases by intranasal administration as prophylactic means.

The preparation was injected in a quantity of 2 drops into each nostril with following massage for spreading preparation in the mucosa. The procedure was repeated twice a day. The curative effect was controlled by the general state of the organism and body temperature.

Example 27

The pharmacological preparation on the basis of a BAA, obtained as in Examples 1-6; 9-14: 16-21 and 23-26, was produced after lyophilic drying of the mentioned BAA in the quantity of 0,001 per cent by mass, with the rest being an excipient. A 10% saccharose solution was used as an excipient. The preparation was used for bee feeding. In comparison with control group, one feeding of a bee family in an amount of 1 liter of the preparation ensures, the following increase:

- survival by 100-160%;
- quantity of flights by 150-200%;
- honey by 35-85%.

Example 28

The pharmacological preparation on the basis of a BAA, obtained as in Examples 1-6: 9-14; 16-21 and 23-26, was produced after lyophilic drying of the mentioned BAA in the quantity of 85.0 per cent by mass, with the rest being an excipient.

A preservative was used as an excipient. After lyophilization a preparation in the form of powder was used for healing open wounds in case of damages traumas, erosions, and burnings of the mucosa. The use of the preparation accelerated regenerative processes approximately by 3-10 times in comparison with control group.

Example 29

The pharmacological preparation on the basis of a BAA. obtained as in Examples 1-6; 9-14; 16-21 and 23-26, was produced in the quantity of 1,0 per cent by mass, with the rest being an excipient. The gel from plant pulp in the form of cucumber-like, apple-like and peach-like puree was used as an excipient. The preparation was used for cosmetic purposes. The curative pharmacological effect controlled by cutaneous elasticity, was shown after 2-6 seances.

Example 30

The pharmacological preparation on the basis of a BAA. obtained as in Examples 1-6; 9-14; 16-21 and 23-26, was produced in the quantity of 1.0 per cent by mass, with the rest being an excipient. The gel based on honey (15 parts), onion juice (3 parts), garlic juice (1 part) and agrimony (burdock) oil (1 part) was used as an excipient. The preparation was used as a mask for strengthening hair-covering of the head. Gel applications to the head hair-covering were made for 2-3 hours. The procedure was repeated once a week. The curative pharmacological effect controlled by the state of head hair-covering was noted after 3-10 seances.

Example 31

The pharmacological preparation on the basis of a $BA_A$. obtained as in Examples 1-6; 9-14; 16-21 and 23-26, was produced in the quantity of 1.0 per cent by mass, with the rest being an excipient. Lanoline mixed with spermaceti in the proportion of 1:3 was used as an excipient. The preparation in the form of an ointment was used for the regeneration and reconstruction of cutaneous covering after dermatosis. The use of the preparation induced 3-6 fold increase of regenerative processes in comparison with control group.

Example 32

The pharmacological preparation on the basis of a BAA, obtained as in Examples 1-6; 9-14; 16-21 and 23-26. was produced in the quantity of 1,0 per cent by mass, with the rest being an excipient. Cacao oil was used as an excipient. For the treatment of endometritis the preparation was used in the form of suppositories. The curative pharmacological effect controlled by the indices of inflammatory reaction, histological studies, blood analysis and some secretions was observed after 10-20 days of usage.

Example 33

The pharmacological preparation on the basis of a BAA, obtained as in Examples 1-6; 9-14; 16-21 and 23-26, was produced in the quantity of 1,0 per cent by mass, with the rest being an excipient. Lanoline mixed with vaseline in the proportion of 1:3 was used as an excipient. The preparation was used in the form of an ointment for treating mucous erosions and in the processes of their regeneration and reconstruction, a 4-8 fold increase of its beneficial effect in comparison with control group was observed.

MATERIALS AND RESEARCH METHODS

Example 34

The amino acid analysis of BAA compound, obtained as in Examples 1-26. was processed in three directions, determining as follows:

- Total amino acid content,
- Amino acid content of peptides and free amino acids in absence of acid insoluble proteins;
- Content of free amino acids.

1) The amino acid analysis of a BAA. obtained as in Examples 1-26 was carried out by hydrolysis of the substance with 6 N hydrochloric acid (6 N HCl) at a temperature of 110°C for 24 hours in vacuum. After the termination of hydrolysis the samples were evaporated on dry sodium hydroxide (NaOH) in vacuum. The dry residue was diluted with didecimolar (0,2 N) sodium citrate buffer pH 2,2 and was put on ionexchange columns of amino acid analyser.

2) For removing acid insoluble proteins 0,5 ml of 3% sulphosalicylic acid were added into 0,5 ml of the sample and allowed to stand for 1 hour, with periodic stirring. The denaturated proteins were removed by centrifugation at 8000 g for 15-20 minutes. The obtained supernatant was subjected to total amino acid analysis.

3) The free amino acid content of a BAA obtained as in Examples 1-26, was determined following sample equilibration with didecinormal (0,2 N) sodium-citrate buffer at pH 2,2 and after filtration through paper filter (with removed ashes) the filtrate was put on the ion exchange columns of amino acid analyser. The amino acid columns were eluted with sodium citrate buffer pH 3, 25; pH 4,25 and pH 5,28.

The fractionated amino acids were treated with ninhydrin reagent and the optical density was determined by flow photometer at the wave length of 440 and 570 nm.

Example 35

The total lipid content of a BAA, obtained as in Examples 1-26, was determined as follows. 0,5 ml of a BAA and 1.5 ml of concentrated sulphuric acid ($H_2SO_4$) were mixed and boiled at a temperature of 95°C for 15 minutes. After cooling, 1.5 ml phosphovanile reagent, (made by "Hemapol" firm. Czechoslovakia) was added to 0.1 ml hydrolyzate according to the well known method of determining total lipides by specific staining (J.M.Knight. S.Anderson. J.M.Rawle.-Clinical Chemistry -1972. -18. - p.199). The optical density of solution was determined at a wave length of 530 nm.

Example 36

The nucleotide content analysis of a BAA, obtained as in Examples 1-26. was carried out as follows.
The acid soluble purine and pyrimidine nucleotides were extracted from 1,5-5.5 ml sample with 5% cooled chloric acid ($HClO_4$). In 20 minutes after the extraction all cooled samples were centrifugated at 2500 g for 10 min., the sediment was washed with 5% chloric acid ($HClO_4$) three times, the supernatant was collected and used for extraction of acid soluble nucleotides.
The acid soluble fraction was hydrolyzed by monomolar chloric acid (1 M $HC1O_4$) at a temperature of 100°C for 1 hour until pyrimidine monophosphates and purine bases were obtained. The solutions were neutralized by potassium hydroxide (KOH) and all products of the hydrolysis were separated in a cation-exchanger "DAUEX" 50x4, using the step elution:

- with distilled water ($H_2O$) - for uric acid and uridinemonophosphate (UMPh);
- didecimolar chloric acid (0,2 M $HClO_4$) - for xanthine and cytosine monophosphate (CMPh);
- tetradecimolar chloric acid (0,4 M $HClO_4$) - for hypoxanthine;

- monomolar chloric acid (1,0 M $HClO_4$) - for guanine;
- dimolar chloric acid (2,0 M $HClO_4$) - for adenine.

Nucleotides were identified by the chromatographical activity in a cation-exchanger comparing with standards, and also by absorption spectrums at a: wave length of 200-300 nm. Nucleotide number was defined, using the coefficient of molar extinction.

Example 37

The immunomodulating activity (properties) of a BAA. obtained as in Examples 1-26, was defined by macrophage activation of peritoneal exudate from Wistar rats in experiments in vitro. The task of these experiments was an increase of enzyme activity in the recovery of nitro-blue tetrazole into diphormazane (NBT-test) and an increase of phagocyte activity by E.Coli.

20 ml of the colourless Hank's solution, consisting of 20 units/ml of heparin, was injected into the abdominal cavity of decapitated animals by syringe.

In 2-3 minutes after abdominal massage the incision was performed, and injected liquid was drained from cavity by the same syringe. Then, after filtration through nylon filter, this liquid was centrifugated at 150-200 g for 15 minutes.

The produced sediment was suspended with semivolumed fresh portion of Hank's solution and recentrifugated under the same conditions.

The 3-4 fold washing of extracted cells was processed, and after that cell concentration of the suspension reached $80 \times 10^6$ cells/ml. The macrophage content of tissue exudate consisted of 25-35% in all cells.

The determination of macrophage enzyme activity in rat peritoneal exudate was performed by spectrophotometry with the recovery of nitro-blue tetrazole in diphormazane. The cell sediment of rat peritoneal exudate was dissolved by the colourless Hank's solution up to the concentration of $80 \times 10^6$ cell/ml.

50 $\mu$l of the sample with pH 7.4 and peptide concentration of 1,0 mg/ml were added into 50 $\mu$l of cell suspension.

This mixture was incubated at a temperature of 37°C for 30 minutes in water bath with permanent shaking. After that 50 $\mu$l of the solution of nitro-blue tetrazole (made by "Reanal" firm (Hungary)), saturated at a temperature of 20°C, was added and incubation was continued for additional 30 minutes.

Then 3 ml of acetone were added into solution with subsequent centrifugation at 2 000 g for 20 minutes. The layer of acetone extract formed over sediment was subjected to photometry at a wave length of 515 nm.

The sample with a preservative based on physiological solution was used for comparing the enzyme activity.

The phagocyte activity of rat peritoneal exudate cells was defined by their ability to E.Coli capture.

50 $\mu$l of the sample with pH 7.4 and peptide concentration of 0,05 mg/ml was added to 50 $\mu$l of cell suspension.

This mixture was incubated at a temperature of 37°C for 30 minutes in water bath with permanent shaking. Finishing incubation the mixture was suspended with 10 ml of the colourless Hank's solution at a temperature of 4°C, with subsequent centrifugating at 150-200 g for 15 minutes.

The cell sediment of rat peritoneal exudate was diluted by Hank's solution up to the concentration of $2.5 \times 10^6$ cells/ml. The produced suspension in an amount of 0,2 ml was put on the glass sized 18 x 18 mm and incubated at a temperature of 37°C for 30 minutes in the atmosphere, comprising 5% carbon dioxide ($CO_2$) for fixing macrophages on glass.

After the incubation, in order to remove some non-fixed cells, the glass was washed in the tumbler with Hank's medium 3 times.

0,2 ml of E.Coli suspension with concentration of $20 \times 10^6$ cells/ml were added to cells remained on the glass and then incubated under similar conditions for 45 minutes. Then the glass was rinsed three times, fixed with methanol and coloured by Romanovsky - Gymze.

The index of phagocytosis (IPh) was defined by the following formula :

$$IPh = (0 + 2.5n + 6t + 9p + 10R)/ \text{ cells number. (1)}$$

where:

0 - cell number, which does not phagocytize E.Coli;
n - cell number, which absorbed 1-4 cells;
t - cell number, which absorbed 5-7 cells;
p - cell number, which absorbed 8-9 cells;
R - cell number, which absorbed 10 and more E.Coli cells.

The identification of macrophages was processed by colouration of unspecific esterase.

Example 38

The immunomodulating activity (properties) of a BAA obtained as in Examples 1-26, was defined by activation of Wistar rat blood neutrophiles in experiments in vitro. The task of these experiments was increasing of enzyme activity of nitro-blue tetrazole recovery into diphormazane (NBT-test) and the increase of phagocyte activity by E.Coli.

The blood from rat tail artery was collected into test-tube with the colourless Hank's solution on heparin and mixed with periodic stirring.

6% dextran T-500 was added to the obtained mixture in a proportion of 3:1 and was placed in the refrigerator for one hour. After erythrocyte sedimentation the leucocyte layer over the sediment was collected by pipette and centrifuged at 250 g for 15 minutes. In order to remove erythrocytes from leucocytic mass the obtained substance was hemolyzed by suspending leucocyte sediment in distilled water with adding Hank's solution and the mixture was centrifuged once more. The sediment of leucocytes was resuspended with fresh Hank's solution and centrifuged under the same conditions. The operation of washing was repeated 3 times.

The definition of enzyme activity of blood neutrophiles was performed by the spectrophotometer with the reduction of nitro-blue tetrazole into diphormazane.

The sediment of rat blood leucocytes was diluted by the colourless Hank's solution up to the concentration of $80 \times 10^6$ cells/ml

100 μl of the cell suspension was added to 300 μl of a BA.A received as in Examples 1-26, with pH 7.4 and peptide concentration up to 1.0 mg/ml.

This mixture was incubated at a temperature of 37°C for 30 minutes in water bath with permanent shaking. After that 400 μl nitro-blue tetrazole solution (made by "Reanal" firm (Hungary)) was added, and all operations were carried out as in Example 37.

The phagocyte activity of leuocytes in rat blood cells was defined by their ability to E.Coli capture.

50 μl of leucocyte suspension was added to 50 μl of a BAA with pH 7,4 and peptide concentration up to 0,05 mg/ml.

This mixture was incubated and, then, 10 ml colourless Hank's solution at a temperature of 4°C was added with subsequent centrifugation at 150-200 g for 15 minutes.

The cell sediment was diluted by 0.3 ml of Hank's solution and added with 0.05 ml E.Coli at a suspension concentration of $500 \times 10^6$ cells/ml. The produced suspension was incubated at a temperature of 37°C for 30 min. After the incubation some smears were done, and after colouring the quantity of phagocyting cells and phagocytosis index (IPh) were measured as in Example 37.

Example 39

The immunomodulating activity (properties) of a BAA, obtained as in Examples 1-26. was defined in experiments on BALB/C mice in vivo. The animals received 2 subcutaneous injections of the preparation at a dose of 0.5 ml/kg of the body weight with two day intervals between injections at peptide concentration of 1,0 mg/ml. The analysis of immunomodulating properties was performed in two hours after last injection. No less than 10 animals were used in each experiment. The animals, treated with 0,08% preservative diluted in physiological solution, were used as control group.

For determining the reaction of lymphocyte blast-trasformation, phitohemaglutenin (PhGA) was used ,as T-cell mitogen and, lypopolysaccharid E.Coli (LPS) was used as B-cell mitogen.

The lymphocytes, extracted from rat spleen, were incubated with these mitogenes at a temperature of 37°C for 72 hours in an atmosphere comprising 5% carbon dioxide ($CO_2$). The level of cell proliferation was defined radiometrically by the index including $^3$H-hymidin. For this purpose $^3$H-hymidin ($3.7 \times 10^4$ Bk/probe) was added to cultures and incubated for 3 hours. Then the samples were put on a millipore filter ("Synpor" N5, Czechoslovakia) with subsequent 3-fold washing including medium 199. 5% solution of trichloroacetic acid (TChA) and ethanol. The filters were dried, put into the bottles with scintillation liquid (LS-106) and the radioactivity of the samples was defined by "Mark-111" counter.

Example 40

The immunomodulating activity (properties) of a BAA, obtained as in Examples 1-26, was defined by the stimulation of natural killer activity in mouse splenocytes in experiments in vivo. The animals received 2 subcutaneous injections of the preparation at a dose of 0.05 ml/kg of the body weight for 2 days at peptide concentration of 1,0 mg/ml. The analysis of immunomodulating properties was performed the next day after last injection. No less than 10 animals were used in each experiment. The animals, treated with 0.08% preservative diluted in physiological solution, were used as control group.

The state of natural killer activity in splenocytes was defined on the YAC-12 mouse lymphoma cells labelled by chrome isotope [51] Cr. The incubation was performed for 18 hours with correlation between target-cells and effector-cells (1 x 10[5]: 2.5 x 10[6] cells) in the proportion of 1:25. The specific release of chrome isotope $^{51}$Cr, corresponding to cytostatic activity of cell-effectors, was calculated by the following formula:

$$^{51}Cr = \frac{(\text{release in an experiment}) - (\text{self-release})}{(\text{maximum release}) - (\text{self-release})} \times 100, \quad (2)$$

where: "maximum release" means - radioactivity, and under such conditions all cells are lysed. The preservative, diluted in physiological solution was used as control for the definition of increased natural killer activity induced by the proposed BASes.

Example 41

The immunomodulating activity of a BAA obtained as in Examples 1-26, was defined by the functional activity of mouse thymus in experiments in vivo under analogical conditions described in Example 39.

The effect was estimated by titre of thymic serum factor (TSF). This method is based on the ability of thymic hormones to restore the sensitivity of spontaneous rosette-forming cells from the spleen of mature mice, to antithymocytic serum, after thymectomy.

The blood serum of experimental animals was ultrafiltrated through the filter of the system CENTRIFLO CF-50A (made by "Amicon" company). 0,1 ml of the undiluted and serially diluted by the medium 199 serum, was used for the reaction. Medium 199 was used as a control.

The mouse spleens were obtained in 10-14 days after thymectomy. The spleen cells were extracted by the method of tissue defibrination with preparative needles in the medium 199. After the filtration through caprone sieve the cells were twice washed with the medium 199 by centrifugation at 4 000 g. The sediment was resuspended in the medium up to the concentration of 4 x 10[7] cells/ml and equilibrium obtained in the volume 0,1 ml, was added to the test - tubes containing serum and medium 199. The consistence of the test - glass was mixed by pipette and incubated at a temperature of 37°C for 60 minutes.

After the incubation 0,1 ml of antithymocyte serum and a complement of the guinea-pig in dilution 1:10, were added to all test-tubes. The suspension was incubated at a temperature of 37°C for additional 30 minutes. Then 0.1 ml of the suspension of rat erythrocytes (12 x 10[7] cells/ml) was added, mixed and centrifuged at 250 g for 5 minutes with subsequent incubation at a temperature of 4-8°C for 60 minutes. The sediment of test - tubes was resuspended for 5 minutes. The rosette calculation was performed in Goriajev's camera. The lymphocyte, binding four and more erythrocytes was considered as a rosette. The last dilution of serum, inducing 50% reduction of the number of rosette-forming cells (RFC) responding to the control was considered as Titre TSF. The results were expressed in the form of logarithm of titre with the basement 2, namely in the form of log A, where A is a titre meaning.

Example 42

The effectiveness of a BAA, produced as in Examples 1-26, manifested in the increase of reparative abilities of rat liver hepatocytes in vivo after subcutaneous injection of carbon tetrachloride ($CCl_4$), was defined by the rehabilitative and reconstructive indices of Na,K-ATPase activity as follows.

Everyday Wistar mature male rats weighting 230-250 g received subcutaneous injection of carbon tetrachloride ($CCl_4$) mixed with vegetable oil in proportion of 1:1 at a dosage of 4.0 ml/kg of the body weight. In 4 hours after the first injection of carbon tetrachloride ($CCl_4$), the experimental animals received the first injection of the preparation at a dose of 0.05 ml/kg of the body weight with one day interval between injections. The efficiency of an agent was registered the next day after last injection. As a control animal liver, analogically damaged by carbon tetrachloride ($CCl_4$), as well as the liver of intact animals untreated with the studied BAA, were used for comparison.

In order to establish the Na, K-ATPase activity, fractions of hepatocyte cell membranes were used.For this purpose, 15 g of cooled liver of three rats were homogenized in 150 g of millimolar borate buffer solution (1MM $Na_2B_4O_7$) with pH=7, 5, and in the presence of quinquedecimillimolar solution of calcium chloride (0,5 MM $CaCl_2$), using the hand glass-teflon homogenizer.

The homogenized mixture was processed by the method of centrifugation at 150 g at a temperature of 4°C for 10-12 minutes. Then, the supernatant was recentrifugated at 100 000 g for 20 minutes, and the obtained sediment was three times washed in 130 ml of buffer solution under similar conditions of centrifugation. After finishing washing the sediment was resuspended in buffer solution, with protein concentration adjusted (according to the method of SDS-Lowry) up to 3,0 mg/ml. The obtained suspension was used in the subsequent analysis.

All operations of the production of membrane fraction were processed under cold conditions.

The Na,K-ATPase activity of hepatocyte plasmatic membranes was defined in the standard medium, comprising:

sixty six millimolar sodium chloride (66 MM NaCl);
thirty four millimolar potassium chloride (34 MM KCl):
quinquemillimolar magnesium chloride (5 MM $MgCl_2$);
twenty five millimolar (25 MM) Tris-HC1, with subsequent adding quinquemillimolar adenosine - triphosphoric acid (5 MM ATPh) before the test.

0,2 ml of the suspension of membrane fraction with protein concentration of 3.0 mg/ml was added to 1,8 ml of the mentioned standard medium and was incubated at a temperature of 37°C for 15 minutes. After that the reaction was stopped and 0,6 ml of 50% solution of trichloroacetic acid (TChA) was added. The mixture was centrifugated at 300-500 g for 15 minutes. To 1.0 ml of the obtained supernatant the following was added: 4 ml of didecimolar (0.2 M) acetate buffer solution with pH=4.0: 0.5 ml of 1% solution of ammonium molybdate in 1% solution of sulphuric acid ($H_2SO_4$); 0.5 ml of ascorbic acid in 0,16% solution of $CuSO_4$.

After mixing all this compounds were incubated at a temperature of 25°C for 10 minutes. The optical density was defined spectrometrically at a wave length of 700 nm.

The activity of Na,K-ATPase was defined by the difference between the activity of general ATPase and Mg-ATPase. In order to define the activity of Mg-ATPase there were processed similar performances, but 0,3 ml of 0,05% solution of strophanthin instead of 0,6 ml of trichloracetic acid (TChA), were added.

It was established that in the toxical damage of liver induced by carbon tetrachloride ($CCl_4$) 3,5-fold decrease in Na, K-ATPase activity was observed while using the present BAA, as in Example 16, its activity can be recovered experimentally no less than by 80% in comparison with the norm. Under the same conditions the well-known hepatotropic preparation "Essentiale"

- an industrial analogue of the present preparation, reconstructs Na, K-ATPase activity by 43.0±4.8%.

Example 43

The effectiveness of a BAA. produced as in Examples 1-26, that manifested in the increase of reparative abilities of rat liver hepatocytes (in vivo) was defined after the subcutaneous injection of carbon tetrachloride ($CCl_4$), according indices of aminotransferase activity in blood and potassium (K) concentration in hepatocytes of the rat liver as follows.

The aminotransferase activity of animal blood, processed as in Example 42, was tested on the test-system of "Hemapol" company (Czechoslovakia) according to the photometrical method with the specific colouring (S.Reitman, S.Frankel.- American Journal of Clinical Pathology,-1957-28-56).

0.05 ml of the serum of rat blood were added to 0.25 ml of the solution, consisting of the following components: decimolar solution of (0,1 M) L-aspartate; didecimolar (0,002 M) 2-oxoglutarate: and decimolar (0,1 M) phosphate buffer solution with pH=7.4. This mixture was incubated at a temperature of 37°C for 60 minutes. After finishing incubation 0.25 ml of the solution consisting of millimolar (0.001M) 2,4-dinitrophenylhydrazine, diluted in monomolar hydrochloric acid (1 M HCl), was added into the solution. The sample was left for 20 minutes at room temperature. After that 2,5 ml of tetradecimolar (0,4 M) dry sodium hydroxide (NaOH) was added, mixed and, after 10 minutes, the optical density at a wave length of 520 nm was measured, comparing with the control solution, where 0.05 ml of physiological solution instead of 0,05 ml of blood serum were added.

It was established that in toxical damage of the liver induced by carbon tetrachloride ($CCl_4$), hepatocyte destruction occured, and, correspondingly, the release of aminotranspherases, with the consequent 2-fold increase of their activity in blood serum is observed

At the same time the usage of the present BAA, obtained as in Example 16, affords the rehabilitation of such activity in the experiments.

Under the same conditions the well-known hepatotropic preparation "Essentiale" reduced the aminotranspherase activity in blood by 87,2-7.4%.

The potassium ($K^+$) concentration in hepatocytes of the liver of experimental rats was defined by the standard method of flame photometry, according to Brikker, using the flame photometer, type FPhM-1 (V.N.Brikker, The definition of K, Na content by the method of the flame photometry. - Laboratornoe delo-1961-7- p.3-6).

It was established, that in the case of toxical damage of liver induced by carbon tetrachloride ($CCl_4$) Na,K-ATPase activity was decreased by 3,5 times, with following decrease in K+ concentration of rat liver hepatocytes by 40%. In the same time the usage of the present BAA, obtained as in Example 16, affords the reconstruction of K+ concentration not less than by 55%. The well known preparation "Essentiale" reduced K+ concentration of hepatocytes by 33,0+6,2% under similar conditions.

Example 44

The stimulation of regenerative processes in case of usage of a BAA produced as in Example 16, and a preparation on its basis, was processed on the experimental modelling of rat ulcer, caused by cryogenic influence.

The upper one third of the abdominal wall of ether anesthetized male rats weighing 190-200 g were subjected to laparatomy with subsequent removal of the stomach and causing its mucous membrane damage by a 10 second cryogenic influence of metallic shaft with a diameter of 7 mm cooled in liquid nitrogen according to the well known method (V.A. Vertelkin. Cryogenic method making an experimental gastric ulcer,-Patologicheskaya fysiologia i eksperimentalnaya terapia., -1987, -N2, - p.77-78). After that the stomach was put back into the abdominal cavity and the wound was sewed. On the 5-th day the animal ulcer, morphologically similar to the human ulcer, appeared and in 3-4 weeks it was healed, without treating

The effectiveness of regenerative abilities of the present BAA and the preparation on its basis, was defined by the acceleration of wound healing process corresponding to the control group of untreated animals, in comparison with the effect, induced by the well known biostimulator "Solcoseryl" (Alkoloid-Skopie company (Jugoslavia), made under the licence of "Solco Basel" (Switzerland)). The biochemical control of regenerative processes by the level of ulcer malonic dialdehyde (MDA) and, pathomorphological examination of some sections of damaged part of the stomach, and measures of damaged surface of the mucosa were performed on the 5-th, 15-th and 25-th days after the operation.

The level of MDA in damaged tissue of the stomach was defined by means of thiobarbituric acid, and its specific concentration corresponding to 1 mg of protein was determined by the known method (Stalnaja I.D., The modern methods in biochemistry, -M., -1977,-p. 66-68).

Its influence on regenerative processes was studied with the use of a preparation, which consisted of a BAA in an amount of 0.1 per cent by mass, and the rest being an excipient, obtained as in Example 16. As an excipient centimolar phosphate buffer solution (0.01 M PBS) pH 7.2 was used.

Starting from the fifth day after operation, when the ulcer was formed, the animals received everyday intramuscular injection for 20 days according to the groups:

1) Control - (0.01 M RBS) at a dose of 0,5 ml/kg:
2) Experiment - present BAA at a dose of 0.5 ml/kg;
3) Experiment - present BAA at a dose of 0.05 ml/kg;
4) Pharmacological analogue - "Solcoseryl" at a dose of 0, 5 ml/kg;

The group of intact animals was used as the standard for comparing the normalization of homeostatic parameters.

During experiments as was notified, a preparation on the basis of the present BAA at a dose of 0.05 ml/kg regenerated the level of MDA by $80\pm12\%$, in comparison with the control group of untreated animals, and stayed higher than that of intact animals by $87\pm1,3\%$, and at a dose of 0,5 ml/kg it normalized the level of MDA up to that of intact animals on the 15-th day.

At the same terms "Solcoseryl" decreases the level of MDA by $38\pm16\%$ and was not effective, staying on the same level as that of control animals, and higher than the level of intact animals by $21\pm9\%$ on the 25-th day. In the experimental groups 2 and 3 the level of MDA was restored up to the norm on the 25-th day.

There were obtained analogical results in the pathomorphological studies. On the 5-th day after the operation the damaged surface of gastric mucosa was equal $13,0\pm1,0$ mm and on the 15th day it decreased to 9,5 mm in control animals.

The frames of damaged zone linked up to this term in the 2nd and 3rd animal groups while they were eroded, and weakly expressed in group 4.

In histological studies the sections from the damaged gastric areas, were weakly expressed, the destructive changes and more early reparative processes were noted in the group 2 comparing with other groups of animals.

The ulcer edges, linked up owing to the vegetation of interstitial tissue and mucosal glandular epithelium were non-characteristic for this area of the mucosa. The defection of mucous membrane was completely exchanged by structurally reconstructed glandular parenchyma, but granular and mature connective tissues were expressed moderately.

Such an effect for "Solcoseryl" was less displayed and destructive deffects were notified in the mucous as well as submucous membranes of pyloroanterior part of the stomach. There were met some zones with the productive inflammatory reaction, riched with cell elements such as fibroblasts, plasmatic cells, histocytes and leucocytes. Similarity between this group of the animals and control group according to these features was noted.

So, the experimental data of such a character point the ability of the present BAA to stimulate regenerative processes, taking into consideration that an efficiency of this BAS considerably exceeds that of industrial analogue - widely known regenerative biostimulator "Solcoseryl".

ANALYSIS OF THE COMPOSITION AND PROPERTIES OF THE PRESENT BAA AND THE PREPARATION MADE ON ITS BASIS

The identification of the present BAA among other biologically active agents can be made directly by biochemical assay and indirectly - by the size of immunomodulating effect which is displayed by the present agent

It was established, that only modified components of cell membranes with modified antigenic structure ensure a considerable increase in immune reaction of the organism in comparison with control. This allows the present BAA to be identified by the following set of indices:

a) An increase in macrophage activity of rat peritoneal exudate, particularly in enzyme activity of macrophages, controlled by NBT-test, no less than by 61% (in the case of nitro-blue tetrazole (Hungarian company "Reanal") use in experiments) an increase in phagocyte activity of E.Coli - no less than by 49%;

b) An increase in activation of rat blood neutrophils, namely an increase in enzyme activity of neutrophils, no less than by 75%, controlled by NBT-test. (in case of nitro-blue tetrazole (Hungarian company "Reanal") use in experiments): an increase in phagocyte activity of E Coli no less than by 62%;

c) An increase in the reaction of blast transformation of T-lymphocytes no less than by 52%, and B-lymphocytes no less than by 69%;

d) An increase in functional activity of the thymus according to the content of the thymic serum factor (TSF) in rat blood no less than by 234%;

e) An increase in natural killer activity of rat splenocytes no less than by 62%;

f) An increase in ability to stabilize plasmatic membranes of rat liver hepatocytes after an injection of carbon tetrachloride ($CCl_4$):

- recovery of Na, K-ATPase activity no less than by 30%;
- recovery of transpherase activity in rat blood no less than by 61%;
- normalization of potassium (K) to sodium (Na) ratio in rat liver hepatocytes no less than by 20%;

The preservative diluted with physiological solution was used as a base for comparison.

This index system of immunomodulating and regenerating properties of the present BAA allows its identification by biological activity as an immunomodulator and stimulator of regenerative processes.

The present BAA and a preparation based on it demonstrate a pronounced effect of the stimulation of regenerative processes and recovery of functional activity of organs and tissues, disturbed in various pathology. Their efficacy exceeds significantly that of the known BAA and preparations of similar administration in the treatment of inflammatory processes.

Thus, the activation of reparative processes of subacute liver damage in experimental modelling hepatitis has a protective effect on plasmatic membranes of hepatocytes with significant diminishing of cytolysis syndrome (following the use of a BAA produced as in Example 16, blood level of aminotranspherases grows no more than by 5% versus 110 to 140% in control).

The activity of membrane-bound enzyme (Na, K-ATPase) restores by 75-90% in comparison with control that considerably normalizes intra- and extracellular ion balance of K/Na. By these indices, the efficacy of the present preparation exceeds that of "Essentiale", a well known hepatotropic preparation, whose indices are 7-20% and 38-48%, respectively (Examples 42 and 43). A preparation on the basis of the present BAA also surpasses Essentiale by its ability to normalize a bilirubin level, indices of lipid metabolism, antitoxic function of the liver.

A preparation on the basis of the Present BAA inhibits the processes of peroxidation of lipides on hepatocyte membranes of intoxicated animals and increases the activity of glutation-dependent antioxidant system in blood, not changing activity of microsomal oxidative enzymes and a cytochrome P-450 content in the liver.

The present BAA considerably activates the reparative as well as regenerative processes, that ensures the restoration of organ mass and substitution of dead cells by proliferating cells from remaining part of the organ.

Thus, in experiment during the activation of regenerative processes in rat liver following partial hepatectomy, there was established that four injections of a preparation on the basis of a BAA, produced as in Example 16, given before the operation with a 2 day intervals, stimulated the transcription processes as well as expressiveness of activation of DNA-polymerase complex of hepatocytic nuclei. The present preparation activates DNA-polymerase B which is responsible for reparation of cell genetic apparatus, and, in the processes of proliferation, stimulated the activity of DNA-polymerase A which is responsible for replication. This affords to increase essentially a rate of cell proliferation and regenerative processes in liver.

The significance of this effect should be appreciated accounting the fact, that such a modelling system is rather strictly determinated genetically and its activation is extremely difficult.

The intracellular processes activated by the present BAA lead to the normalization of functional indices in pathological processes, however without disturbation of functional state and stucture of the liver in healthy animals.

This phenomenon is based on significant essential influence of immune system of the organism and, first of all, the system of mononuclear phagocytes (SMPh) with the leading role of macrophages in the realization of regenerative processes. In the liver, such macrophages are Kupffer's cells controlling regenerative and reparative processes of the organ.

Several injections of the preparation were sufficient for their stimulation. On the second day, the generalized reaction of all SMPh cells, and, first of all, Kupffer's cells was noted. It was revealed by an increase in removing foreign substances from blood stream. There were also noted an increase in antibacterial activity and the phagocytosis completion of peritoneal macrophages and blood neutrophils, which was very important in the treatment and prophylaxis of bacterial infections as well as inflammatory processes.

The effect of the stimulation of regenerative processes by the present BAA and by preparations on its basis, can be also displayed in the treatment of ulcers, wounds of cutaneous covering.

In this respect, their effectiveness exceeds the analogical indices of a commercial analogue, such as a well known biostimulator "Solcoseryl" (Example 44).

The present agent and a preparation on its basis administered to animals in cases of inflammatory processes revealed positive results. Thus, in the treatment of proliferative inflammation induced experimentally on Selye's modelling of "pocket" granuloma and modified by Rosen (Technique for the reproduction of standard aseptic inflammation, -Patologitcheskaya Fisiologa i Experimentalnaya Terapia -1961, -5 No 6, page 72-76), a preparation on the basis of the present BAA, suppressed the formation of granulation fibrotic tissue and of exudate twice effective as Solcoseryl.

In the treatment of cow mastitis, recovery was noted in 80-93% cases, while using a well known BAAs as well as (in case of using) antibiotics combined with antiinflammatory preparation Mastysan, induced recovery in no more than 70% cases: and in spontaneous healing the recovery was noted in 53-63% cases of the controls. In the treatment of calf pneumonia the following results were obtained: the present BAA. ensured the recovery in 85-95% cases; known BAAs induced recovery in 69% cases; antibiotics plus Boviverex induced recovery in 60-70% cases; in control group recovery was noted in 48-61% cases.

In the treatment of cow endometritis, with the present BAA the recovery was observed in 80-95% cases, while treatment with known BAAs as well as antibiotics plus Oxytocin, induced recovery in no more than 70% cases.

The immunomodulating and regenerating properties of the known and present BAAs were assessed experimentally in accordance with the set of indices identifying experimental objects. The results of experiment are given in Table 6. showing the quantitative indices of the effects of the well known both BAA No 1. obtained according to the technology, described in (US-A-4455302) and BAS No 2, represented on the basis of the data published in (EP-A-0318030), and the present BAA represented in the form of groups WS, PM, TS, LM and CW, namely generalized by the results:

WS - Examples 1-4 (components of cell membranes);
PM - Examples 5-6 (components of plasmatic membranes);
TS - Examples 9-14 (thermostable components of cell membranes);
LM - Examples 16-19 (low-molecular components of cell membranes M≤10.0 kDa ;
CW - Examples 20-21 (carbohydrates containing low - molecular components of cell membranes).

A physiological solution of sodium chloride with a preservative was used as the control. The results obtained allowing comparison between the present and known BAAs are summarized in Table 6.

These results are dealt with the characteristics of the present BAA produced in the form of a mixture with undetermined structure. The findings which are necessary for its identification are also represented.

The analysis of findings given in Table 6 allows the identification of the present BAA and assessment of its biological activity by the presence of components of cell membranes with modified structure, in its composition whose effects were not revealed in known BAAs.

Table 6

| Immunomodulating and regenerating effects of the biologically active agents (BAAs) | | Quantitative indices of BAA effects | | | | | | |
|---|---|---|---|---|---|---|---|---|
| | | Well known | | The present BAA | | | | |
| | | No.1 | No.2 | WS | PM | TS | LM | CW |
| 1. An increase in the activity of macrophages of rat peritoneal exudate versus control, % | Enzyme activity (NBT-test) | 46,8 ±4,3 | | 70,2 ±8,9 | 93, 6 ±8,3 | 102,3 ±15, 5 | 172,8 ±28, 7 | 212,4 ±12,3 |
| | Phagocyte activity according to E.Coli | 33,4 ±4,3 | | 61,4 ±12,1 | 81,7 ±9,2 | 92,1 ±14,4 | 153,6 ±31,8 | 186,3 ±14,6 |
| 2. An increase in the activity of blood neutrophyles versus control, % | Enzyme activity (NBT-test) | 67,2 ±3,8 | | 84,3 ±9,4 | 105,2 ±10,7 | 116,7 ±14,6 | 148,7 ±19,7 | 171,3 ±10,3 |
| | Phagocyte activity by E.Coli | 44,2 ±5,7 | | 61,1 ±9,0 | 72,3 ±6,1 | 76,7 ±9,2 | 103,2 ±17,4 | 121,4 ±7,8 |
| 3. Stimulation of blasttransformation reaction versus control, % | T-lymphocytes | 42,3 ±4,6 | | 58,6 ±6.2 | 75,4 ±8,1 | 83,2 ±17,4 | 131,7 ±20,3 | 176,1 ±15,8 |
| | B-lymphocytes | 46,3 ±4,7 | | 77,6 ±8, 1 | 101,4 ±17,4 | 118,8 ±18,4 | 192,4 ±28,5 | 243,6 ±18,9 |
| 4. An increase in titre of thymic serum-like factor (TSF) versus control, % | | 117,8 ±9,1 | | 310,6 ±76,7 | 412,7 ±84, 5 | 496,4 ±70,1 | 764,3 ±94, 6 | 948,7 ±58,9 |
| 5. Stimulation of natural killer activity of rat splenocytes versus control,% | | 53,8 ±5,4 | 62,0 ±30,0 | 71,8 ±9,7 | 88,3 ±10,6 | 101,4 ±12,2 | 143,6 ±26,4 | 197,1 ±15,1 |
| 6. An increase in reparative abilities of rat hepatocytes by reconstruction of activity after CCl$_4$ injection[4] versus control. % | Na, K-ATP-ases | 22,6 ±3,4 | | 35,4 ±5,1 | 51,2 +6,2 | 56,3 ±7,1 | 86,2 ±7,8 | 95,7 ±4,3 |
| | Blood aminotranspherases | 23,4 ±4,8 | | 71,6 ±10,3 | 85,3 ±7,1 | 91,4 ±4,6 | 96,5 ±3,5 | 98,1 ±1,9 |
| | K$^+$ Concentration of hepatocytes | 14,2 ±3,1 | | 25,4 ±4,7 | 36,8 ±5,8 | 41,2 ±5,4 | 66,7 ±8,7 | 84,3 ±9,2 |
| Differences are significant with control P<0,05 | | | | | | | | |

Additional analysis of findings in Table 6, allows to conclude, that immunomodulating effect of the present BAA is significantly increased in cases, comprising components of plasmatic cell membranes (BAA-PM), components of cell membranes in the form of thermostable components (BAa-ST), the low-molecular components (BAA-LM) and in the form of carbohydrate-containing components (BAA-CW). The growth of mentioned effect is directed from the left to right.

The properties of the present BAA and a preparation on its basis are in close dependence on its composition.

According to the biochemical analysis (Table 7), the present BAA includes in general the following organic compounds: polypeptides, peptides, amino acids, bound and free carbohydrates, lipids, nucleotides and other organic compounds. The findings in Table 7 show the distribution of the organic compounds by mass, when cattle embryonic tissue is used as starting raw material. Besides, in Table 7 are shown some varieties of the present BAA, such as WS, PM, TS, LM, CW, produced by the present method in all its versions.

The findings in Tables 6 and 7 allows to make comparative analysis of the results of realization of the present and known methods of producing BAAs as well as their compositions and properties.

A characteristic feature of the present BAA is the presence of carbohydrate-contaning components in its composition which determine significantly antigen structure of cell membranes of the tissue in the processes of its modification.

That is why the content of carbohydrates in the most active fractions (fractions 3, 4 and 5 in Table 4) of low-molecular components of cell membranes with a molecular mass M = 0.8-10.0 kDa (oligocarbohydrates, glycopeptides, glycolipids, nucleotides) is the decisive factor for identification and characterization of the present BAA. In this respect, the ratio between carbohydrate content and that of peptides within the components with a molecular mass of 0.8-10.0 kDa is a critical index which is determined by the formula:

$$G = \frac{Cc}{Cp} \qquad (3).$$

Table 7

| Organic compounds | Content of organic compounds in present BAA mass % | | | | | | |
|---|---|---|---|---|---|---|---|
| | Known BAAs | | present BAA | | | | |
| | No. 1 | No. 2 | WS | PM | TS | LM | CW |
| Polypeptides | 32,1 ±6,1 | 43,5 ±6,5 | 36,5 ±8,0 | 33,0 ±7,5 | 20,5 ±6,8 | less than 0,3 | |
| Peptides | 16,3 ±3,2 | - | 18,0 ±3,5 | 18,5 ±3,3 | 23,4 ±3,1 | 30,5 ±2,8 | 32,3 ±3,7 |
| Amino acids | 31,7 ±6,2 | - | 15,0 ±2,0 | 15,0 ±2,1 | 19,6 ±3,0 | 24,0 ±7,5 | less 0,5 |
| **TOTAL**: | 80,1 ±5,1 | 43,5 ±6,5 | 69,5 ±6,0 | 66,5 ±5,1 | 63,5 ±6,8 | 54,8 ±7,5 | 33,1 ±3,7 |
| Content of carbohydrates -high-molecular compounds with a molecular mass M>10.0 kDa | 1,9 ±0,8 | 56,5 ±6,5 | 3,0 ±2,0 | 1,5 ±0,8 | 0,9 ±0,5 | less than 0,2 | |
| -low-molecular compounds with a molecular mass M=0.8 10,0 kDa | 3,0 ±0,5 | - | 15,5 ±4,0 | 20,5 ±4,8 | 18,7 ±3,7 | 24,9 ±5,9 | 49,7 ±8,3 |
| -tree monomers or compounds with a molecular mass M<0.8 kDa | 10,0 ±2,7 | - | 4,5 ±1,0 | 4,0 ±2,5 | 8,9 ±1,6 | 7,3 ±1,2 | 14,0 ±2,1 |
| **IN ALL**: | 14,9 ±2,7 | 56,5 ±6,5 | 23,0 ±7,0 | 26,0 ±6,8 | 28,5 ±5,8 | 32,4 ±5,5 | 63,9 ±6,1 |
| Lipides, nucleotides and other organic compounds | 5,0 ±1,6 | - | 7,3 ±3,0 | 7,5 ±3,1 | 8,0 ±2,6 | 12,8 ±4,2 | 3,0 ±2,5 |
| **TOTAL**: | 100,0 | 100,0 | 100,0 | 100,0 | 100,0 | 100,0 | 100,0 |
| Ratio between carbohydrate and peptide contents, G* | 0.18 | - | 0,86 | 1,11 | 0,80 | 0,82 | 1,54 |
| Calculated efficiency, K | 0,17 | - | 0,63 | 0,82 | 0,91 | 1,60 | 2,04 |
| Weight of dry residue of the BAA, mg/ml | 32,6 ±9,6 | - | 9,3 ±2,2 | 8,7 ±2,0 | 7,2 ±1,8 | 5,2 ±1,6 | 3,5 ±0,9 |
| Weight of preservative mg/ml | - | - | 0,7 | 0,7 | 0,7 | 0,7 | 0,7 |

(continued)

| Organic compounds | Content of organic compounds in present BAA mass % | | | | | | |
| | Known BAAs | | present BAA | | | | |
| | No. 1 | No. 2 | WS | PM | TS | LM | CW |
| BAA concentration in pow der (composition) mass % | - | - | 93,0 ±1,3 | 92,6 ±1,3 | 91,1 ±1,7 | 88,1 ±2,6 | 83,3 ±3,3 |

Differences are significant with control P<0.05
\* Values are the mean of determinations as indicated $\pm$ SEM where Cc and Cp - are concentrations of carbohydrates and peptides, within compounds with a molecular mass M = 0.8-10.0 kDa respectively.

For the present BAA value of this index is in the range of Gd=0.6 2.0, whereas it does not exceed Gwk<0.25 for known $BA_{As}$.

Thus, a value of the index G characterizes the properties of the hydrolysis products and depends on the raw material used in the present method, upon the complex of operations processing the mentioned raw material, and upon the regimens of hydrolysis.

It should be noted, that the effectiveness of a desired product depends on temperature - time characteristics of hydrolysis and such dependence can be represented graphically (see the drawing). The curves 1, 2 and 3 schematically illustrate changes in effectiveness of the present BAA depending on the duration of hydrolysis and its temperature T $(T_1>T_2>T_3)$.

As seen in Curve 2, the effectiveness of the present BAA grows from E=0 to E=$R_{max}$ within the time interval from t=0 to t=$t_2$. It can be explained by an increase in the proportion of low-molecular carbohydrate-containing components of the desired product with a concurrent decrease in the proportion of high-molecular, immunoinhibiting and ballast compounds in the process of hydrolysis.

In the time interval from t=$t_2$ up to t=$t_4$, the effectiveness falls down from E=$R_{max}$ up to E=0 as a consequence of the fact, that the prolongation of hydrolysis process results in changes of structure of carbohydrates- containing components with a molecular mass M = 0.8-10.0kDa, and first of all, glycopeptides, in connection with hydrolyzing their peptide basis linking several oligocarbohydrate chains. Modified oligocarbohydrates and inactive monomer forms of carbohydrates also appear in hydrolyzate.

The above described processes are temperature-dependent and have a pronounced maximum which in total reflects the maxima of corresponding curves 1, 2 and 3, namely rate of speed in the pointed processes, and, respectively, the constants of rate of Speed in the mentioned chemical reactions strongly depend on the value of the working temperature T in the pointed range. Within these limits, there is ensured that the functional activity of enzymes, which are necessary for the hydrolysis, are retained. When the temperature T increases, maxima of the curves 1, 2 and 3 move to the left, in the direction of coordinate beginning. Therefore, the choice of the working temperature of hydrolysis, should be accompanied by the choice of the time interval of hydrolysis namely such a time- interval, in which the meaning of effectiveness E should be no less than 0,8 $R_{max}$ :

$$E > 0,8 \ R_{max} \qquad\qquad (4)$$

The chosen raw material, duration of hydrolysis, and working temperature of hydrolysis, as well as hydrolysis stopping at a predetermined moment ensure a higher effectiveness of the present BAA comparing with the known BAAs.

Besides, the mentioned conditions allow reproduction of the results to be ensured, namely, they allow a standardization of the present BAA with predetermined properties.

Immunomodulating properties of the present BAA particularly its capability to increase the activity of the macrophages of rat peritoneal exudate in vitro, depend on temperature-time characteristics of hydrolysis process at working temperature ranging from T=4-45°C which was established experimentally by the method of production of the present BAA. This indicates a need for multiple evaluation of quality of the present BAA. The choice of the working temperature within the pointed range is performed accounting the following:

- at a temperature of T<4°C, the duration of hydrolysis increases considerably, which can lower economical effectiveness of the present method.

- at a temperature of T>45°C. there is a reduction in activities of enzymes ensuring hydrolyzation of the raw material and an acceleration in the processes of decomposition.of active components of the desired product. As a result, deterioration of the quality of desired product and a reduction in economical efficiency of the present method were observed.

It should be noted, that the efficiency of the present BAA depends largely not only on the presence of necessary active components in its composition, but also on the composition of the desired product that was established experimentally.

The main contribution to the immunomodulating effect of the present BAA was made by carbohydrate-containing components of the fractions 3, 4 and 5 (Table 4) with their molecular mass of M = 0.8-10.0 kDa(oligocarbohydrates, glycopeptides, glycolipids, nucleotides). Later they will be referred to as oligocarbohydrates (OCH) . However, it should be taken into consideration, that the influence of the mentioned oligocarbohydrates on immunomodulating activity of the present BA A has complex nature and correlates with several effects. Thus, it was established empirically that the index R which reflects the effectiveness, is generally expressed by the following equation:

$$R = F \times (A + B + C) \qquad (5),$$

where F - is the coefficient which describes the degree of the modification of cell membrane components of animal tissue used as raw material:

A, B, C - are concentrations of ingredients in the BAA composition, where:

A - is relative concentration of carbohydrates, within components with molecular mess M = 0.8-10.0 kDa (OCH), in respect to all the other ingredients of a BAA:

$$A = \frac{OCH}{PP+P+Ama+PCH+OCH+MCH+Gl+L+N} \qquad (6);$$

B - is relative concentration of 0CH in respect to ingredients with a molecular mass M>0.8 kDa;

$$B = \frac{OCH}{PP + P + PCH + OCH + Gl} \qquad (7);$$

C - is relative concentration of OCH in respect to ingredients with a molecular mass M>10.0 kDa .

$$C = \frac{OCH}{PP + PCH + OCH} \qquad (8).$$

where the concentration of BAA ingredients is expressed as:

OCH - oligocarbohydrates, that is carbohydrates within components with a molecular mass M=0.8-10.0 kDa;
PCH - polycarbohydrates, that is the carbohydrates within components with a molecular mass M>10.0 kDa;
MCH - monocarbohydrates, that is carbohydrates in the form of monomers or components with a molecular mass M<0.8 kDa;
PP - polypeptides with a molecular, mass M>10.0 kDa;

P - peptides with a molecular mass M>10.0 kDa;
AMA - free amino acids;
GL - glycolipids;
L - lipids;
N - nucleotides.

According to the equation 5, the value of the index R depends not only on the nature of the raw material used for the BAA production and characterized by the coefficient F, but also on the method of producing a BAA, which determines a quantitative value of the sum:

$$A + B + C = K. \qquad (9)$$

Then the equation 5 takes a form:

$$R = F \times K, \qquad (10)$$

where K - is the coefficient Characterising the effectiveness Of the present BAA produced by the present method.

As can be seen from the equations 3 to 8, the index K, to a great extent, depends on the concentration of OCH, PP and PCH. However, relative contribution of these ingredients to the BAS specificity is not unidirectional and the value of the coefficient K increases with concentration of OCH and decreases with concentration of PP and PCH.

At last, from the analysis of equations 3 to 8, it can be concluded, that the most significant relative improvement in immunomodulating properties and effects of the present BAA is ensured in certain specific cases of the realization of the present method of producing this BAA It can be explained by the fact that the concentration of OCH in the present BAA increases, while that of high-molecular compounds in the form of PP and PCH decreases at each next stage of realization of the present method producing a BAA.

Such data correlate with the results of analysis of compositions of the present BAA and known BAAs , which are illustrated in Table 7.

As seen from Tables 2 and 7, the index K of the present BAA is significantly higher than that of known BAAs which correlates with contents of OCH, PP and PCH.

Thus, the concentration of OCH is 3.0%, while the concentration of PP + PCH is 34.0% where the index K is 0.17 for the known BAA No 1 (US-A-4455302) in Table 7. As for the present BA A, these values are 11.5%-30.8%, 39.5% and 0.47-1.90, respectively. For the present BAA, produced from carbohydrate-containing compounds, these values are 49.7%, 0.5%, 2.20, respectively (Table 2).

Thus, the higher technical result, achieved in the present method for producing BAA', is in causal dependence on the totality of its significant signs.

Thus, the immunomodulating properties of the present $BA_A$. as follows from the analysis of Tables 2 and 7, are considerably higher than those of known BAAs . The analysis of the data allows the conclusion that the effectiveness R of the present BAA correlates with the value of the index K of desired products produced by the different variants of the present method when using the same raw material and, consequently, with the same value of the coefficient F. This conclusion is confirmed by the results of experiments (Tables 1 and 6).

Thus, enzyme activity of macrophages in rat peritoneal exudate, according to the NBT-test (see Table 6) is higher, as compared with control by 70% for water-soluble components of the present BAA, and by 170% for low-molecular components, that is the technical result, as judged from specific activity, increases in 2.4 times. Similarly, the technical result increases in 2.5 times by the phagocytic activity of macrophages. These data are in good correlation with values of the coefficient K for each of these products, which differed in 2.6 times.

Thus, the comparatively higher technical result, related to specific activity of the present BAA, is connected with the totality of its indices as cause and effect. And so, in all cases the specific activity of the present BAA rises with the increase of its indices of realization (in Table 6 - from the left to the right).

INDUSTRIAL USE

The invention which relates to a BAA , a method of its production and a pharmacological preparation on the basis of said BAA, are based on the selection of initial raw materials, the choice of conditions for the processing and obtaining a BAA and a preparation with guaranteed high specific activity. The chosen technology for obtaining a BAA ensures the reproductivity of results, that affords to standardize obtaining of the present BAA with desired properties, namely, with given fixed immunomodulating activity. This activity defines the pharmacological, therapeutical and generally biological effects in cases of

According to the character of their biological influence, the obtained desired products in general and particular cases realizing the present method for obtaining the BAA are equal, but they are different by their specific activity. Thus the advisable use of this or another concrete biologically active agent is determined by the requirements of medicine and/or veterinary. In this connection, the selection of a certain BAA from others is determined by the correlation between demands to its immunomodulating activity and the effectiveness and the cost (economy) of its production in every specific case of usage of the desired product. Particularly the high efficiency of an agent comprising low-molecular components of the present BAA-LM with a molecular mass M≤10.0 kDa as well as carbohydrate-containing components of BAA-CW is completely displayed in the form of increased activity of macrophages, neutrophiles, lymphocytes, natural killers, which ensures the strengthening of the regenerative processes (Table 6). For agricultural animals such effects are followed by additional increase of animal weight in the case of intensive fattening (Table 8). In the treatment of respiratory and infectious diseases (parainfluenza) such an advantage is not significant and so the use of a BAA, comprising water-soluble components of cellular membranes of both BAA-WS and BAA-PM will be more economical. A substance, comprising thermostable components of the present BAA-TS should be used in the treatment of inflammatory processes (mastitis, endometritis and so on). Preparing preparations for external use in the form of ointments, gels, compresses, suppositories, intranasal inhalations or animal drinks the use of a BAA, comprising thermostable components of BAA-TS instead of a BAA-LM will be more economical.

In order to obtain maximum effect the present BAA and a preparation on its basis would be more advisable to use for the activation and stimulation of regenerative processes and rehabilitation of functional activity of the organs and tissues in different pathology, and also in the treatment of inflammatory processes, where its effectiveness can considerably exceed the efficiency of the well known BASes and analogically administered preparations.

In addition, the present BAA and preparations on its basis are more effective substances in the treatment of infectious diseases (parainfluenza. ARD, and so on). Their using induce recovery of younger animals of horned cattle in 88-95% cases, while the known BAA as well as antibiotics and the preparation "Boviverex", induce the recovery of animals in not more than 70% cases, and in the spontaneous recovery of control group - within the limits of 58-67% cases.

The present BAA increases the resistence of animals to infectious diseases, that considerably reduces the mortality level of the cattle population.

The utilization of this BAA as a prophylactic preparation reduced calve morbidity associated with parainfluenza up to 5% from the total number, while the application of the well known BAAs reduced the morbidity of animals up to 12-17%, and in control group this index was reduced up to 22-28%.

The normalization of general biological state of agricultural animals utilizing the present BAA improves productive indices, particularly rises an additional increase in living weight, without lowering meat quality and simultaneously nearing these indices to genetically programmed level in the case of intensive fattening.This feature distinguishes the present BAA and preparations on its basis from hormonal and other growth stimulators, rising quantitative indices with negative influence on the animal homeostasis and reducing quality of the products.

In the case of intensive fattening, the administration of the present BAA and preparations on its basis to animal organisms, with the decline of the homeostasis parameters from the norm, ensures the rising of additional weight increase on average by 52-145% comparing with control group without the use of the present BAA, while using well known BAA analogues (of the substance No 1) induced additional increase of weight in comparison with the average increase by 12-24% of control group under similar conditions. The control and experimental groups of horned cattle consisted of 14-18 calves of the same age, initial weight and etiology with decline from the norm.

The normalization of homeostatic parameters promotes an increase in:

- milk yields of the horned cattle, and small cattle;
- egg - laying of the poultry;
- fur quality of the fur-bearing animals;
- survival of younger animals;
- honey of bees and so on.

The present BAA and a preparation on its basis are non-toxical, did not cause side effects (allergies and so on).

The present preparation causes increased stimulation of regenerative processes, namely, rehabilitation of the activity of the liver tissue in case of hepatitis and liver regeneration after partial hepatectomy, healing of ulcers, wounds and cutaneous surface in case of damages and wounds, reconstruction of blood formula in case of great blood loss and in other cases.

The comparative data of pharmacological, therapeutical and general biological effects of utilizing the present BAA and a preparation on its basis in animals are shown in Table 8. The

Table 8

| Pharmaceutical. therapeutical and general biological effects of BAA | | | Quantitative characteristics | | | |
|---|---|---|---|---|---|---|
| | | | Known BAAs | Present BAA | | |
| | | | No.1 | WS | TS | LM |
| 1.Inflamma tory processes | Mastitis of cows, recovery % | Exp. Control | 70±5 61±5 | 80±5 58±8 | 86±5 63±7 | 93±5 53±8 |
| | Pneumonia recovery % | Exp. Control | 69±6 55±7 | 85±5 51±8 | 92±6 61±6 | 95±5 48±6 |
| 2. Infectious diseases | Para-in fluenza & ARD*, recovery % | Exp. Control | 70±6 64±7 | 88±6 58±6 | 94±6 61±7 | 95±5 67±5 |
| 3. Additional increase in calf weight versus cont rol, % | | | 24±7 | 54±8 | 70±12 | 145±31 |
| 4.Acute toxicity LD ml/kg (mg/kg) of body weight | | | more than 25 (260) | more than 25 (260) | | |
| Differences are significant with control P<0,05 * ARD - acute respiratory diseases analysis of data of this table illustrates, that a biological activity of the present BAA considerably exceeds biological activity of analogues. | | | | | | |

Thus, the present BAA and a preparation on its basis, have a wide spectrum of abilities for their use in veterinary and medicine and can be used for the normalization of the physiological state of the organism in treatment of diseases, controlled by the immune system.

The administration of the present BAA in the form of the present preparation affords stimulation of:

- functional activity of liver tissue in hepatitis;
- regeneration of liver after hepatectomy;
- healing of ulcers, wounds, cutaneous surface in wounds and damages;
- reducing of blood formula in cases of wounds and damages;
- treatment of inflammatory diseases (mastitis, pneumonias, endometritis);
- treatment of infectious diseases (parainfluenza, ARD);
- increase of the animal resistence to infectious and inflammatory processes;
- improvement of general biological state of the organism;
- rise of additional weight increase of animals.

The practical use of the present BAA confirms its non-toxicity and absence of negative side effects - allergy, delayed hypersensitivity.

The absence of side effects in a BAA used and a pharmacological preparation on its basis, and data of its pharmacological, therapeutical and general biological effects on animal organism determined the possibility of clinical approbation of a BAA and its preparations.

Clinical examination of patients receiving the present BAA revealed positive influence of a preparation on physiological state of the organism, as well as practically full analogy with the preclinical experiments on the animals.

The method of production of the present BAA can be realized in the laboratory, and industrial conditions as well.

THE SOURCES OF INFORMATION TAKEN INTO CONSIDERATION

1. FR 24 13 912, A61K 35/44, 10.01.1978;

- Bontemps R. - The medicinal preparations based on an embryon and the method of their production.

2. US 44 55 302, A61K 37/00, 18.03.1982;

- H.J. Robertson - Medical protein hydrolysate, process of making the same and processes of utilizing the protein hydrolysate to aid in healing traumatized areas.

3. SU 1 412 596, A61K 39/00, 23.07.1988;

- Masakazu Adachi (Japan) - The method of producing lymphocytes cytotoxic against cancer cells and process of preparing glycosidic linkage related antigen (Rus.).

4. EP 0 106 285, A61K 39/00, 37/02, 25.04.1984;

- Otsuka Pharmaceutical Co., Ltd. (Japan) - Masakazu Adachi - Glycosidic linkage related antigen, process for producing the same and anticancer agent containing the same as effective components.

5. EP 0 157 427, A61K 37/02, 09.10.1985;

- Otsuka Pharmaceutical Co., Ltd. (Japan) - Masakazu Adachi - Process for preparing glycosidic linkage related antigen.

6. EP O 318 030, A61K 37/02, 26.11.1987;

- Otsuka Pharmaceutical Co., Ltd. (Japan) - Masakazu Adachi - Glycoprotein, preparation method for pharmaceutical compositions comprising it and use.

7. USSR Author's Certificate 904 707, A61 35/26, 35/28, 1982; - A.M. Smirnov et al. (Leningrad Veterinary Institute) - The substance for increasing organism resistance of the younger animals and process for preparing (Rus.).
8. H.M. Berman, W. Gram, M.A. Spirtes - Biochemica et Biophysica Acta, 1969, V. 183, 1, pg. 10-18.
9. J. Gurd, W. Evans, H. Perkins - Biochemistry Journal, 1973 , V. 135, 4, pg. 827-832.
10. G. Schapira, J. Dobocz - Biochemica et Biophysica Acta, 1974, V. 345, 3, pg. 348-358.
11. G.M. Higgins, R.M. Anderson - Experimental pathology of the liver - Arch. Pathology, 1931, No. 12, pg. 186.
12. U.K. Laemmli - Nature, 1970, V. 227, No. 5259, pg. 680-685.
13. S. Seifter, S. Dayton, C. Hovic - Arch. Biochemistry and Biophysics, 1950, 25, 1, pg. 191-200.
14. J.M. Knight, S. Anderson, J.M. Rawle - Clinical Chemistry, 1972, 18, pg. 199.
15. S. Reitman, S. Frankel - American Journal of Clinical Pathology, 1957, 28, 56.
16. B.H. Brikker - The definition of K, Na contents by the method of flame photometry - Laboratornoye Delo, 1961, 7, pg. 3-6 (Rus.).
17. Vertelkin V.A. - Cryogenic method of making an experimental gastric ulcer - Pat. fiziol. i experim. ter., 1987, No. 2, pg. 77-78 (Rus.).
18. Stalnaya I.D., The modern methods of biochemistry, M. 1977, pg. 66-68 (Rus.).
19. Rosen V.B. - The method of the reproduction of the standard aseptic inflammation - Pat. fiziol. i experim. ter., 1961, 5, No. 6, pg. 72-76 (Rus.).

**Claims**

1. A biologically active agent having immunomodulating properties which contains thermostable, low-molecular, modified components of cell membranes of homogenized animal tissue, said components being organic compounds, being thermostable at a temperature of up to 120°C, having a molecular mass $M \leq 10.0$ kDa and being isolated from cell membranes of cells, which have been taken from the processes of embryogenesis and/or proliferation and/or differentiation of the cells, or pathology preceding and/or accompanying the process of regeneration and/or reparation of the tissue, said agent being obtainable by disintegrating and homogenizing raw material of animal tissue, said raw material being selected from the group comprising embryonic tissue except tissue of human embryos, placental tissue, differentiating thymocytes of the thymus, spermatogones and spermatocytes of the testes, epithelial tissue of the intestine, bone marrow cells, regenerating cutaneous tissue, regenerating hepatic tissue, tissue of regenerating amphibian limbs, pathologically transformed tissue taken prior to the stage of regeneration, or any combination thereof, purifying homogenate from non-disintegrated tissue elements by means of filtration and/or centrifugation at 150 to 250 g for 10 to 15 min, isolating the sediment comprising cell membranes from said homogenate, hydrolyzing said sediment, filtering and/or centrifugating the hydrolysis products at above 1.500 g for 20 to 40 min, and collecting the supernatant of the hydrolysis products comprising cell membrane components as desired product.

2. The agent according to Claim 1, **characterized in that** said thermostable, low-molecular components of cell membranes comprise components of plasmatic cell membranes.

3. The agent according to Claim 1, **characterized in that** said low-molecular components of cell membranes have a molecular mass M = 0.8 - 10.0 kDa.

4. The agent according to Claim 2, **characterized in that** said low-molecular components of plasmatic cell membranes have a molecular mass M = 0.8 - 10.0 kDa.

5. The agent according to Claim 3, **characterized in that** said low-molecular components of cell membranes comprise carbohydrate-containing components.

6. The agent according to Claim 4, **characterized in that** said low-molecular components of plasmatic cell membranes comprise carbohydrate-containing components.

7. The agent according to any one of Claims 1 to 6, **characterized in that** it contains organic compounds at the following ratio in percent by mass:

| | |
|---|---|
| Peptides | from 28.0 to 60.0; |
| Amino acids | from 0.5 to 31.5; |
| Carbohydrates | from 16.0 to 70.0; |
| Lipids, nucleotides and other organic compounds | from 0.5 to 17.0. |

8. The agent according to Claim 7, **characterized in that** it contains carbohydrates at the following ratio in percent by mass within:

| | |
|---|---|
| High-molecular compounds having a molecular mass M>10.0 kDa | up to 3.0; |
| Low-molecular compounds having a molecular mass M=0.8-10.0 kDa | from 11.5 to 57.9; |
| Free monomers having a molecular mass M<0.8 kDa | from 3.5 to 17.0. |

9. The agent according to any one of Claims 1 to 6, **characterized in that** the ratio between the mass content of carbohydrates and that of peptides, within said organic compounds having a molecular mass M=0.8-10.0 kDa, ranges from 0.6 to 2.0.

10. The agent according to any one of Claims 1 to 6, **characterized in that** it contains organic compounds at the following ratio in percent by mass:

| | |
|---|---|
| Peptides | from 40.0 to 60.0; |
| Amino acids | from 12.9 to 17.1; |
| Carbohydrates | from 16.0 to 32.8; |
| Lipids, nucleotides and other organic compounds | from 4.4 to 10.6. |

11. The agent according to any one of Claims 1 to 6, **characterized in that** it contains organic compounds at the following ratio in percent by mass:

| | |
|---|---|
| Peptides | from 34.0 to 50.1; |
| Amino acids | from 16.6 to 22.6; |

(continued)

| Carbohydrates | from 22.7 to 34.2; |
| Lipids, nucleotides and other organic compounds | from 5.4 to 10.6. |

**12.** The agent according to any one of Claims 3 to 6. **characterized in that** it contains organic compounds at the following ratio in percent by mass:

| Peptides | from 28.0 to 33.6; |
| Amino acids | from 16.5 to 31.5; |
| Carbohydrates | from 6.9 to 37.9; |
| Lipids, nucleotides and other organic compounds | from 8.6 to 17.0. |

**13.** The agent according to any one of Claims 5 to 6, **characterized in that** it contains organic compounds at the following ratio in percent by mass:

| Peptides | from 29.0 to 36.3; |
| Amino acids | less than 0.5; |
| Carbohydrates | from 57.7 to 70.0; |
| Lipids, nucleotides and other organic compounds | from 0.5 to 5.5. |

**14.** A method for producing a biologically active agent having immunomodulating properties according to claim 1, wherein the method comprises disintegrating and homogenizing raw material of animal tissue except tissue of human embryos, purifying homogenate from non-disintegrated tissue elements by means of filtration and/or centrifugation at 150 to 250 g for 10 to 15 min, isolating the sediment comprising cell membranes from said homogenate, hydrolyzing said sediment, filtering and/or centrifugating the hydrolysis products at above 1.500 g for 20 to 40 min, and collecting the supernatant of the hydrolysis products comprising cell membrane components as desired product.

**15.** The method according to Claim 14, **characterized in that** said raw material is selected from the group comprising embryonic tissue, placental tissue, differentiating thymocytes of the thymus, spermatogones and spermatocytes of the testes, epithelial tissue of the intestine, bone marrow cells, regenerating cutaneous tissue, regenerating hepatic tissue, tissue of regenerating amphibian limbs, pathologically transformed tissue taken prior to the stage of regeneration, or any combination thereof,

**16.** The method according to Claim 14, **characterized in that** said sediment comprising cell membranes is isolated from the purified homogenate by means of centrifugation at 1.500 to 90.000 g and/or filtration through a filter with a characteristic pore size less than 1 $\mu$m with subsequent washing by means of resuspending and repeatedly centrifugating and/or filtering up to obtaining the sediment comprising cell membranes in an amount of no less than 60 percent by mass in relation to organic compounds represented in said sediment.

**17.** The method according to Claim 16, **characterized in that** said sediment comprising cell membranes is subjected, prior to hydrolysis, to additional centrifugation at 20.000 to 100.000 g in density gradient for 1 to 2 hours with isolating plasmatic cell membranes.

**18.** The method according to Claim 14, **characterized in that** said hydrolysis is performed at a temperature ranging from 4°C to 45°C.

**19.** The method according to Claim 14, **characterized in that** said hydrolysis is performed in the presence of a preservative.

**20.** The method according to any one of Claims 14 to 9, **characterized in that** said supernatant of the hydrolysis products comprising cell membrane components is subjected to thermal treatment at a temperature ranging from 60°C to 120°C till complete denaturation of thermolabile compounds, followed by additional filtration and/or centrifugation at more than 1.500 g for 20 to 40 min with collecting supernatant comprising thermostable components of cell membranes as desired product.

21. The method according to any one of Claims 14 to 19, **characterized in that** said hydrolysis products comprising cell membrane components are subjected, prior to filtration and/or centrifugation, to thermal treatment at a temperature ranging from 60°C to 120°C till complete denaturation of thermolabile compounds.

22. The method according to any one of Claims 14 to 19, **characterized in that** said supernatant of the hydrolysis products comprising cell membrane components is subjected to fractionation by means of dialysis through a semipermeable membrane and/or ultrafiltration and/or gelfiltration with collecting a fraction comprising low-molecular components having a molecular mass M ≤ 10.0 kDa as desired product.

23. The method according to any one of Claims 14 to 19, **characterized in that** said hydrolysis products comprising cell membrane components are subjected to fractionation by means of dialysis through a semipermeable membrane and/or ultrafiltration and/or gelfiltration with collecting a fraction comprising iow-molecular components having a molecular mass M ≤ 10.0 kDa as desired product.

24. The method according to Claim 20, **characterized in that** said supernatant of the hydrolysis products comprising components of cell membranes is subjected to fractionation by means of dialysis through a semipermeable membrane and/or ultrafiltration and/or gelfiltration with collecting a fraction comprising low-molecular components having a molecular mass M ≤ 10.0 kDa as desired product.

25. The method according to Claim 22, **characterized in that** said fraction comprising low-molecular components of cell membranes is subjected to affinity chromatography on lectines with collecting a fraction comprising carbohydrate-containing components as desired product.

26. The method according to Claim 24, **characterized in that** said fraction comprising low-molecular components of cell membranes is subjected to affinity chromatography on lectines with collecting a fraction comprising carbohydrate-containing components as desired product.

27. A preparation for the normalization of the physiological state of human and animal organisms, which contains a biologically active agent having immunomodulating properties according to claim 1 in an amount of 0.001 to 85.0 percent by mass with the rest being an excipient.

28. The preparation according to Claim 27, **characterized in that** said biologically active agent comprises organic compounds at the following ratio in percent by mass:

| | |
|---|---|
| Peptides | from 28.0 to 60.0; |
| Amino acids | from 0.5 to 31.5; |
| Carbohydrates | from 16.0 to 70.0; |
| Lipids, nucleotides and other organic compounds | from 0.5 to 17.0. |

29. The preparation according to Claim 28, **characterized in that** said biologically active agent comprises organic compounds at the following ratio in percent by mass:

| | |
|---|---|
| Peptides | from 40.0 to 60.0; |
| Amino acids | from 12.9 to 17.1; |
| Carbohydrates | from 16.0 to 32.8; |
| Lipids, nucleotides and other organic compounds | from 4.4 to 10.6. |

30. The preparation according to Claim 28, **characterized in that** said biologically active agent comprises organic compounds at the following ratio in percent by mass:

| | |
|---|---|
| Peptides | from 34.0 to 50.1; |

(continued)

| | |
|---|---|
| Amino acids | from 16.6 to 22.6; |
| Carbohydrates | from 22.7 to 34.2; |
| Lipids, nucleotides and other organic compounds | from 5.4 to 10.6. |

**31.** The preparation according to Claim 28, **characterized in that** said biologically active agent comprises organic compounds at the following ratio in percent by mass:

| | |
|---|---|
| Peptides | from 26.0 to 33.6; |
| Amino acids | from 16.5 to 31.5; |
| Carbohydrates | from 20.9 to 37.9; |
| Lipids, nucleotides and other organic compounds | from 8.6 to 17.0. |

**32.** The preparation according to Claim 28, **characterized in that** said biologically active agent comprises organic compounds at the following ratio in percent by mass:

| | |
|---|---|
| Peptides | from 29.0 to 36.3; |
| Amino acids | less than 0.5; |
| Carbohydrates | from 57.7 to 70.0; |
| Lipids, nucleotides and other organic compounds | from 0.5 to 5.5. |

**33.** The preparation according to any one of Claims 30 to 32, **characterized in that** said excipient is a physiological solution of sodium chloride and/or a saline buffer solution and/or carbohydrate compounds and/or fats of minerals, animals, vegetables or synthetic origin or any combination thereof.

**34.** The preparation according to any one of Claims 28 to 33, **characterized in that** it additionally contains a preservative.

**35.** Use of a biologically active agent having immunomodulating properties according to claim 1 for the manufacture of a preparation for the normalization of the physiological state of human and animal organisms, said preparation being suitable for administration to said organisms and consisting of said biologically active agent in an amount ranging from 0.001 to 85.0 percent by mass with the rest being an excipient.

**36.** The use according Claim 35, **characterized in that** said preparation being suitable for administration as intramuscular injections at a dose ranging from 0.005 to 0.5 mg/kg of the body weight with 1 to 7 day intervals between injections and/or inhalations at a dose ranging from 0.012 to 0.12 mg/kg of the body weight with 4 to 48 hour intervals between inhalations and/or sublingual administrations at a dose ranging from 0.012 to 0.12 mg/kg of the body weight with 4 to 48 hour intervals between administrations and/or wet feedings at a dose ranging from 0.02 to 1.0 mg/kg of the body weight with 1 to 10 day intervals between feedings and/or oral pills at a dose ranging from 0.02 to 1.0 mg/kg of the body weight with 1 to 10 day intervals between administrations and/or intranasal administrations at a daily dose ranging from 0.001 to 0.05 mg/kg of the body weight with 2 to 24 hour intervals between administrations till the normalization of physiological parameters.

**37.** The use according to Claim 35, **characterized in that** said preparation being suitable for administration as applications to mucous or wound surfaces in the form of compresses, suppositories, ointments, powders and gels with 1 to 10 day intervals between applications till the normalization of physiological parameters.

**Patentansprüche**

**1.** Biologisch wirksamer Stoff mit immunmodulierenden Eigenschaften enthaltend thermostabile, niedermolekulare, modifizerte Komponenten von Zellmembranen homogenisierten tierischen Gewebes, wobei die Komponenten organische Verbindungen darstellen mit einer molekularen Masse von M ≤ 10,0 kDa, die bei einer Temperatur von bis zu 120°C thermostabil sind und aus den Zellmembranen von Zellen isoliert sind, die aus Prozessen der Embryogenese und/oder Proliferation und/oder Differenzierung der Zellen und/oder einer Pathologie, welche dem Prozeß der Regeneration und/oder Reparation von Gewebe vorausgeht und/oder diesen begleitet, stammen, wobei der Stoff erhältlich ist durch Desintegrieren und Homogenisieren von Rohmaterial aus tierischem Gewebe, wobei das Rohmaterial ausgewählt ist aus der Gruppe, enthaltend embryonales Gewebe ausgenommen Gewebe menschlicher Embryonen, Placentagewebe, differenzierende Thymocyten der Thymusdrüse, Spermatogonien und Spermatocyten der Hoden, Epithelgewebe des Darms, Knochenmarkzellen, regenerierendes Hautgewebe, regenerierendes hepatisches Gewebe, Gewebe von regenerierenden Amphibienextremitäten, pathologisch verändertes Gewebe, das vor dem Stadium der Regeneration entnommen wurde und jede Kombination davon, Reinigen des Homogenats von nicht desintegrierten Gewebeelementen durch Filtrieren und/oder Zentrifugieren bei 150 - 250 g für 10 - 15 min, Isolieren des Komponenten von Zellmembranen enthaltenden Sediments aus dem Homogenat, Hydrolyse dieses Sediments, Filtrieren und/oder Zentrifugieren der Hydrolyseprodukte bei über 1500 g für 20 bis 40 min und Sammeln des Überstandes der Hydrolyseprodukte enthaltend Komponenten von Zellmembranen als Zielprodukt.

2. Stoff nach Anspruch 1, **dadurch gekennzeichnet, daß** die thermostabilen, niedermolekularen Komponenten von Zellmembranen Komponenten von plasmatischen Zellmembranen enthalten.

3. Stoff nach Anspruch 1, **dadurch gekennzeichnet, daß** niedermolekularen Komponenten von Zellmembranen eine molekulare Masse von M = 0,8 - 10,0 kDa aufweisen.

4. Stoff nach Anspruch 2, **dadurch gekennzeichnet, daß** die niedermolekularen Komponenten von plasmatischen Zellmembranen eine molekulare Masse von M = 0,8 -10,0 kDa aufweisen.

5. Stoff nach Anspruch 3, **dadurch gekennzeichnet, daß** niedermolekularen Komponenten von Zellmembranen kohlenhydratenthaltende Komponenten umfassen.

6. Stoff nach Anspruch 4, **dadurch gekennzeichnet, daß** niedermolekularen Komponenten von plasmatischen Zellmembranen kohlenhydratenthaltende Komponenten umfassen.

7. Stoff nach einem der Ansprüche 1 - 6, **dadurch gekennzeichnet, daß** er organische Verbindungen bei folgendem Verhältnis in Masseprozent enthält:

| | |
|---|---|
| Peptide | 28,0 bis 60,0; |
| Aminosäuren | 0,5 bis 31,5; |
| Kohlenhydrate | 16,0 bis 70,0; |
| Lipide, Nucleotide und andere organische Verbindungen | 0,5 bis 17,0. |

8. Stoff nach Anspruch 7, **dadurch gekennzeichnet, daß** er die Kohlenhydrate bei folgendem Verhältnis in Masseprozent enthält:

| | |
|---|---|
| hochmolekulare Verbindungen mit einer molekularen Masse M>10.0 kDa | bis 3,0; |
| niedermolekulare Verbindungen mit einer molekularen Masse M=0.8-10.0 kDa | von 11,5 bis 57,9; |
| Freie Monomere mit einer molekularen Masse M<0.8 kDa | von 3,5 bis 17,0. |

9. Stoff nach einem der Ansprüche 1 - 6, **dadurch gekennzeichnet, daß** innerhalb der organischen Verbindungen mit einer molekularen Masse von M=0,8-10,0 kDa das Verhältnis zwischen dem Massegehalt an Kohlenhydraten und dem an Peptiden 0,6 bis 2,0 beträgt.

10. Stoff nach einem der Ansprüche 1 - 6, **dadurch gekennzeichnet, daß** er organische Verbindungen bei folgendem Verhältnis in Masseprozent enthält:

| Peptide | 40,0 bis 60,0; |
| Aminosäuren | 12,9 bis 17,1; |
| Kohlenhydrate | 16,0 bis 32,8; |
| Lipide, Nucleotide und andere organische Verbindungen | 4,4 bis 10,6. |

**11.** Stoff nach einem der Ansprüche 1 - 6, **dadurch gekennzeichnet, daß** er organische Verbindungen bei folgendem Verhältnis in Masseprozent enthält:

| Peptide | 34,0 bis 50,1; |
| Aminosäuren | 16,6 bis 22,6; |
| Kohlenhydrate | 22,7 bis 34,2; |
| Lipide, Nucleotide und andere organische Verbindungen | 5,4 bis 10,6. |

**12.** Stoff nach einem der Ansprüche 3-6, **dadurch gekennzeichnet, daß** er organische Verbindungen bei folgendem Verhältnis in Masseprozent enthält:

| Peptide | 28,0 bis 33,6; |
| Aminosäuren | 16,5 bis 31,5; |
| Kohlenhydrate | 6,9 bis 37,9; |
| Lipide, Nucleotide und andere organische Verbindungen | 8,6 bis 17,0. |

**13.** Stoff nach einem der Ansprüche 5 - 6, **dadurch gekennzeichnet, daß** er organische Verbindungen bei folgendem Verhältnis in Masseprozent enthält:

| Peptide | 29,0 bis 36,3; |
| Aminosäuren | unter 0,5; |
| Kohlenhydrate | 57,7 bis 70,0; |
| Lipide, Nucleotide und andere organische Verbindungen | 0.5 bis 5.5. |

**14.** Verfahren zur Herstellung des biologisch wirksamen Stoffs mit immunmodulierenden Eigenschaften nach Anspruch 1, umfassend Desintegrieren und Homogenisieren von Rohmaterial aus tierischem Gewebe, ausgenommen Gewebe menschlicher Embryonen, Reinigen des Homogenats von nicht desintegrierten Gewebeelementen durch Filtrieren und/oder Zentrifugieren bei 150 - 250 g für 10 - 15 min, Isolieren des Komponenten von Zellmembranen enthaltenden Sediments aus dem Homogenat, Hydrolyse dieses Sediments, Filtrieren und/oder Zentrifugieren der Hydrolyseprodukte bei über 1500 g für 20 bis 40 min und Sammeln des Überstandes der Hydrolyseprodukte enthaltend Komponenten von Zellmembranen als Zielprodukt.

**15.** Verfahren nach Anspruch 14, **dadurch gekennzeichnet, daß** das Rohmaterial ausgewählt ist aus der Gruppe, enthaltend embryonales Gewebe, Placentagewebe, differenzierende Thymocyten der Thymusdrüse, Spermatogonien und Spermatocyten der Hoden, Epithelgewebe des Darms, Knochenmarkzellen, regenerierendes Hautgewebe, regenerierendes hepatisches Gewebe, Gewebe von regenerierenden Amphibienextremitäten, pathologisch verändertes Gewebe, das vor dem Stadium der Regeneration entnommen wurde und jede Kombination davon.

**16.** Verfahren nach Anspruch 14, **dadurch gekennzeichnet, daß** das Komponenten von Zellmembranen enthaltende Sediment von dem gereinigten Homogenat abgetrennt wird durch Zentrifugieren bei 1500 bis 90000 g und/oder Filtrieren durch einen Filter mit einer charakteristischen Porengröße von unter 1 μm und anschließendes Waschen durch Resuspendieren und wiederholtes Zentrifugieren und/oder Filtrieren bis zum Erhalt eines Sediments enthaltend nicht unter 60 Masseprozent Komponenten von Zellmembranen bezogen auf die im Sediment enthaltenen organischen Verbindungen.

17. Verfahren nach Anspruch 16, **dadurch gekennzeichnet, daß** das Komponenten von Zellmembranen enthaltende Sediment vor der Hydrolyse einer zusätzlichen Dichtegradientenzentrifugation bei 20000 bis 100000 g unterworfen wird unter Isolieren von plasmatischen Zellmembranen.

18. Verfahren nach Anspruch 14, **dadurch gekennzeichnet, daß** die Hydrolyse bei einer Temperatur von 4°C bis 45°C durchgeführt wird.

19. Verfahren nach Anspruch 14, **dadurch gekennzeichnet, daß** die Hydrolyse in Gegenwart eines Konservierungsmittels durchgeführt wird.

20. Verfahren nach einem der Ansprüche 14 - 19, **dadurch gekennzeichnet, daß** der Überstand der Hydrolyseprodukte enthaltend Komponenten von Zellmembranen einer thermischen Behandlung bei einer Temperatur von 60 bis 120°C bis zur vollständigen Denaturierung von thermolabilen Verbindungen unterworfen wird, gefolgt von zusätzlichem Filtrieren und/oder Zentrifugieren bei über 1500 g für 20 bis 40 min und Sammeln des Überstandes enthaltend thermostabile Komponenten von Zellmembranen als Zielprodukt.

21. Verfahren nach einem der Ansprüche 14 - 19, **dadurch gekennzeichnet, daß** die Hydrolyseprodukte enthaltend Komponenten von Zellmembranen vor dem Filtrieren und/oder Zentrifugieren einer thermischen Behandlung bei einer Temperatur von 60 bis 120°C bis zur vollständigen Denaturierung von thermolabilen Verbindungen unterworfen werden.

22. Verfahren nach einem der Ansprüche 14 - 19, **dadurch gekennzeichnet, daß** der Überstand der Hydrolyseprodukte enthaltend Komponenten von Zellmembranen einer Fraktionierung durch Dialyse durch eine semipermeable Membran und/oder Ultrafiltration und/oder Gelfiltration unterworfen wird unter Sammeln einer Fraktion enthaltend niedermolekulare Komponenten mit einer molekularen Masse M ≤ 10.0 kDa als Zielprodukt.

23. Verfahren nach einem der Ansprüche 14 - 19, **dadurch gekennzeichnet, daß** Hydrolyseprodukte enthaltend Komponenten von Zellmembranen einer Fraktionierung durch Dialyse durch eine semipermeable Membran und/oder Ultrafiltration und/oder Gelfiltration unterworfen wird unter Sammeln einer Fraktion enthaltend niedermolekulare Komponenten mit einer molekularen Masse M ≤ 10.0 kDa als Zielprodukt.

24. Verfahren nach Anspruch 14, **dadurch gekennzeichnet, daß** der Überstand der Hydrolyseprodukte enthaltend Komponenten von Zellmembranen einer Fraktionierung durch Dialyse durch eine semipermeable Membran und/ oder Ultrafiltration und/oder Gelfiltration unterworfen wird unter Sammeln einer Fraktion enthaltend niedermolekulare Komponenten mit einer molekularen Masse M ≤ 10.0 kDa als Zielprodukt.

25. Verfahren nach Anspruch 22, **dadurch gekennzeichnet, daß** die Fraktion enthaltend niedermolekulare Komponenten von Zellmembranen einer Lektin-Affinitätschromatographie unterworfen wird unter Sammeln einer Fraktion enthaltend Kohlenhydratenthaltende Komponenten als Zielprodukt.

26. Verfahren nach Anspruch 24, **dadurch gekennzeichnet, daß** die Fraktion enthaltend niedermolekulare Komponenten von Zellmembranen einer Lektin-Affinitätschromatographie unterworfen wird unter Sammeln einer Fraktion enthaltend Kohlenhydratenthaltende Komponenten als Zielprodukt.

27. Präparat zur Normalisierung des physiologischen Zustands des menschlichen und tierischen Organismus, enthaltend einen biologisch wirksamen Stoff mit immunmodulierenden Eigenschaften nach Anspruch 1 in einer Menge von 0,001 bis 85,0 Masseprozent, wobei der Rest auf einen Excipient entfällt.

28. Präparat nach Anspruch 27, **dadurch gekennzeichnet, daß** der biologisch wirksame Stoff organische Verbindungen bei folgendem Verhältnis in Masseprozent enthält:

| | |
|---|---|
| Peptide | 28,0 bis 60,0; |
| Aminosäuren | 0,5 bis 31,5; |

(fortgesetzt)

| | |
|---|---|
| Kohlenhydrate | 16,0 bis 70,0; |
| Lipide, Nucleotide und andere organische Verbindungen | 0,5 bis 17,0. |

**29.** Präparat nach Anspruch 28, **dadurch gekennzeichnet, daß** der biologisch wirksame Stoff organische Verbindungen bei folgendem Verhältnis in Masseprozent enthält:

| | |
|---|---|
| Peptide | 40,0 bis 60,0; |
| Aminosäuren | 12,9 bis 17,1; |
| Kohlenhydrate | 16,0 bis 32,8; |
| Lipide, Nucleotide und andere organische Verbindungen | 4,4 bis 10,6. |

**30.** Präparat nach Anspruch 28, **dadurch gekennzeichnet, daß** der biologisch wirksame Stoff organische Verbindungen bei folgendem Verhältnis in Masseprozent enthält:

| | |
|---|---|
| Peptide | 34,0 bis 50,1; |
| Aminosäuren | 16,6 bis 22,6; |
| Kohlenhydrate | 22,7 bis 34,2; |
| Lipide, Nucleotide und andere organische Verbindungen | 5,4 bis 10,6. |

**31.** Präparat nach Anspruch 28, **dadurch gekennzeichnet, daß** der biologisch wirksame Stoff organische Verbindungen bei folgendem Verhältnis in Masseprozent enthält:

| | |
|---|---|
| Peptide | 26,0 bis 33,6; |
| Aminosäuren | 16,5 bis 31,5; |
| Kohlenhydrate | 20,9 bis 37,9; |
| Lipide, Nucleotide und andere organische Verbindungen | 8,6 bis 17,0. |

**32.** Präparat nach Anspruch 28, **dadurch gekennzeichnet, daß** der biologisch wirksame Stoff organische Verbindungen bei folgendem Verhältnis in Masseprozent enthält:

| | |
|---|---|
| Peptide | 29,0 bis 36,3; |
| Aminosäuren | unter 0,5; |
| Kohlenhydrate | 57,7 bis 70,0; |
| Lipide, Nucleotide und andere organische Verbindungen | 0,5 bis 5,5. |

**33.** Präparat nach einem der Ansprüche 30 bis 32, **dadurch gekennzeichnet, daß** der Excipient eine physiologische Lösung aus Natriumchlorid und/oder eine Kochsalzpufferlösung und/oder Kohlenhydratverbindungen und/oder Fette von Mineralien, Tieren, Pflanzen oder synthetischer Herkunft oder jede Kombination davon darstellt.

**34.** Präparat nach einem der Ansprüche 28 bis 33, **dadurch gekennzeichnet, daß** es zusätzlich ein Konservierungsmittel enthält.

**35.** Verwendung eines biologisch wirksamen Stoffs mit immunmodulierenden Eigenschaften nach Anspruch 1 zur Herstellung eines Präparats zur Normalisierung des physiologischen Zustands des menschlichen und tierischen Organismus, wobei das Präparat für die Verabreichung an den Organismen geeignet ist und den biologisch wirksamen Stoff in einer Menge von 0,001 bis 85,0 Masseprozent enthält, wobei der Rest auf einen Excipient entfällt.

**36.** Verwendung nach Anspruch 35, **dadurch gekennzeichnet, daß** das Präparat geeignet ist zur Verabreichung als intramuskuläre Injektionen bei einer Dosierung von 0,005 bis 0,5 mg/kg des Körpergewichts in Intervallen von 1 bis 7 Tagen zwischen den Injektionen und/oder als Inhalationen bei einer Dosierung von 0,012 bis 0,12 mg/kg des Körpergewichts in Intervallen von 4 bis 48 Stunden zwischen den Inhalationen und/oder als sublinguale Gabe bei einer Dosierung von 0,012 bis 0,12 mg/kg des Körpergewichts in Intervallen von 4 bis 48 Stunden zwischen den Gaben und/oder als flüssige Verabreichung bei einer Dosierung von 0,02 bis 1,0 mg/kg des Körpergewichts in Intervallen von 1 bis 10 Tagen zwischen den Gaben und/oder als oral zu verabreichende Pillen bei einer Dosierung von 0,02 bis 1,0 mg/kg des Körpergewichts in Intervallen von 1 bis 10 Tagen zwischen den Gaben und/oder als intranasale Verabreichung bei einer Tagesdosis von 0,001 bis 0,05 mg/kg des Körpergewichts in Intervallen von 2 bis 24 Stunden zwischen den Gaben bis zur Normalisierung der physiologischen Parameter.

37. Verwendung nach Anspruch 35, **dadurch gekennzeichnet, daß** das Präparat geeignet ist zur Verabreichung auf Schleimhäuten oder Wundoberflächen in Form von Kompressen, Suppositorien, Salben, Pulvern und Gelen in Intervallen von 1 bis 10 Tagen zwischen den Verabreichungen bis zur Normalisierung der physiologischen Parameter.

## Revendications

1. Agent biologiquement actif ayant des propriétés d'immunomodulation qui contient des composants modifiés, de faible masse moléculaire, thermostables de membranes cellulaires de tissu animal homogénéisé, lesdits composants étant des composés organiques, étant thermostables à une température jusqu'à 120°C, ayant une masse moléculaire $M \leq 10,0$ kDa et étant isolés des membranes cellulaires des cellules, qui ont été prélevés à partir des procédés d'embryogenèse et/ou de prolifération et/ou de différenciation des cellules, ou d'une pathologie précédant et/ou accompagnant le processus de régénération et/ou de réparation du tissu, ledit agent pouvant être obtenu en désintégrant et en homogénéisant une matière première de tissu animal, ladite matière première étant choisie parmi le groupe comprenant le tissu embryonnaire à l'exception du tissu des embryons humains, le tissu placentaire, les thymocytes différentiateurs du thymus, les spermatogonies et les spermatocytes des testicules, le tissu épithélial de l'intestin, les cellules de la moelle osseuse, le tissu cutané de régénération, le tissu hépatique de régénération, le tissu de membres d'amphibiens de régénération, le tissu transformé pathologiquement prélevé avant le stade de régénération, ou une quelconque combinaison de ceux-ci, en purifiant l'homogénat issu d'éléments de tissu non désintégrés au moyen d'une filtration et/ou d'une centrifugation à 150 à 250 g pendant 10 à 15 min., en isolant le sédiment comprenant les membranes cellulaires issues dudit homogénat, en hydrolysant ledit sédiment, en filtrant et/ou en centrifugeant les produits d'hydrolyse à plus de 1 500 g pendant 20 à 40 min., et en collectant le surnageant des produits d'hydrolyse comprenant les composants de membranes cellulaires comme produit désiré.

2. Agent selon la revendication 1, **caractérisé en ce que** lesdits composants de faible masse moléculaire thermostables de membranes cellulaires comprennent des composants de membranes cellulaires plasmatiques.

3. Agent selon la revendication 1, **caractérisé en ce que** lesdits composants de faible masse moléculaire de membranes cellulaires ont une masse moléculaire $M = 0,8 - 10,0$ kDa.

4. Agent selon la revendication 2, **caractérisé en ce que** lesdits composants de faible masse moléculaire de membranes cellulaires plasmatiques ont une masse moléculaire $M = 0,8 - 10,0$ kDa.

5. Agent selon la revendication 3, **caractérisé en ce que** lesdits composants de faible masse moléculaire de membranes cellulaires comprennent des composants contenant des hydrates de carbone.

6. Agent selon la revendication 4, **caractérisé en ce que** lesdits composants de faible masse moléculaire de membranes cellulaires plasmatiques comprennent des composants contenant des hydrates de carbone.

7. Agent selon l'une quelconque des revendications 1 à 6, **caractérisé en ce qu'**il contient des composés organiques au taux qui suit en pour cent en masse :

| | |
|---|---|
| Peptides | de 28,0 à 60,0 ; |
| Acides aminés | de 0,5 à 31,5 ; |
| Hydrates de carbone | de 16,0 à 70,0 ; |

(suite)

Lipides, nucléotides et autres composés organiques     de 0,5 à 17,0.

**8.** Agent selon la revendication 7, **caractérisé en ce qu'**il contient des hydrates de carbone au taux qui suit en pour cent en masse dans :

Composés de masse moléculaire élevée ayant une masse moléculaire $M > 10,0$ kDa     jusqu'à 3,0 ;

Composés de faible masse moléculaire ayant une masse moléculaire $M = 0,8 - 10,0$ kDa     de 11,5 à 57,9 ;
Monomères libres ayant une masse moléculaire $M < 0,8$ kDa     de 3,5 à 17,0.

**9.** Agent selon l'une quelconque des revendications 1 à 6, **caractérisé en ce que** le rapport entre la teneur en masse d'hydrates de carbone et celle de peptides, dans lesdits composés organiques ayant une masse moléculaire $M = 0,8 - 10,0$ kDa, va de 0,6 à 2,0.

**10.** Agent selon l'une quelconque des revendications 1 à 6, **caractérisé en ce qu'**il contient des composés organiques au taux qui suit en pour cent en masse :

Peptides     de 40,0 à 60,0 ;
Acides aminés     de 12,9 à 17,1 ;
Hydrates de carbone     de 16,0 à 32,8 ;
Lipides, nucléotides et autres composés organiques     de 4,4 à 10,6.

**11.** Agent selon l'une quelconque des revendications 1 à 6, **caractérisé en ce qu'**il contient des composés organiques au taux qui suit en pour cent en masse :

Peptides     de 34,0 à 50,1 ;
Acides aminés     de 16,6 à 22,6 ;
Hydrates de carbone     de 22,7 à 34,2 ;
Lipides, nucléotides et autres composés organiques     de 5,4 à 10,6.

**12.** Agent selon l'une quelconque des revendications 3 à 6, **caractérisé en ce qu'**il contient des composés organiques au taux qui suit en pour cent en masse :

Peptides     de 28,0 à 33,6 ;
Acides aminés     de 16,5 à 31,5 ;
Hydrates de carbone     de 6,9 à 37,9 ;
Lipides, nucléotides et autres composés organiques     de 8,6 à 17,0.

**13.** Agent selon l'une quelconque des revendications 5 à 6, **caractérisé en ce qu'**il contient des composés organiques au taux qui suit en pour cent en masse :

Peptides     de 29,0 à 36,3 ;
Acides aminés     moins de 0,5 ;
Hydrates de carbone     de 57,7 à 70,0 ;
Lipides, nucléotides et autres composés organiques     de 0,5 à 5,5.

**14.** Procédé pour produire un agent biologiquement actif ayant des propriétés d'immunomodulation selon la revendication 1, dans lequel le procédé comprend de désintégrer et d'homogénéiser une matière première de tissu animal à l'exception du tissu d'embryons humains, de purifier l'homogénat issu des éléments de tissu non désintégrés au moyen d'une filtration et/ou d'une centrifugation à 150 à 250 g pendant 10 à 15 min., d'isoler le sédiment comprenant des membranes cellulaires issues dudit homogénat, d'hydrolyser ledit sédiment, de filtrer et/ou de centrifuger les produits d'hydrolyse à plus de 1 500 g pendant 20 à 40 min., et de collecter le surnageant des produits d'hydrolyse comprenant les composants de membranes cellulaires comme produit désiré.

15. Procédé selon la revendication 14, **caractérisé en ce que** ladite matière première est choisie parmi le groupe comprenant le tissu embryonnaire, le tissu placentaire, les thymocytes différentiateurs du thymus, les spermatogonies et les spermatocytes des testicules, le tissu épithélial de l'intestin, les cellules de la moelle osseuse, le tissu cutané de régénération, le tissu hépatique de régénération, le tissu de membres d'amphibiens de régénération, le tissu transformé pathologiquement prélevé avant le stade de régénération, ou une quelconque combinaison de ceux-ci.

16. Procédé selon la revendication 14, **caractérisé en ce que** ledit sédiment comprenant les membranes cellulaires est isolé de l'homogénat purifié au moyen d'une centrifugation à 1 500 à 90 000 g et/ou d'une filtration à travers un filtre avec une taille de pores caractéristique inférieure à 1 $\mu$m avec lavage subséquent au moyen d'une remise en suspension et d'une centrifugation répétée et/ou d'une filtration jusqu'à obtenir le sédiment comprenant les membranes cellulaires dans une quantité de pas moins de 60 pour cent en masse par rapport aux composés organiques représentés dans ledit sédiment.

17. Procédé selon la revendication 16, **caractérisé en ce que** ledit sédiment comprenant les membranes cellulaires est soumis, avant hydrolyse, à une centrifugation additionnelle à 20 000 à 100 000 g dans un gradient de densité pendant 1 à 2 heures avec isolation des membranes cellulaires plasmatiques.

18. Procédé selon la revendication 14, **caractérisé en ce que** ladite hydrolyse est effectuée à une température dans une plage de 4°C à 45°C.

19. Procédé selon la revendication 14, **caractérisé en ce que** ladite hydrolyse est effectuée en présence d'un conservateur.

20. Procédé selon l'une quelconque des revendications 14 à 19, **caractérisé en ce que** ledit surnageant des produits d'hydrolyse comprenant les composants de membranes cellulaires est soumis à un traitement thermique à une température allant de 60°C à 120°C jusqu'à dénaturation complète des composés thermolabiles, suivi d'une filtration et/ou d'une centrifugation additionnelles à plus de 1 500 g pendant 20 à 40 min. avec collecte du surnageant comprenant les composants thermostables de membranes cellulaires comme produit désiré.

21. Procédé selon l'une quelconque des revendications 14 à 19, **caractérisé en ce que** lesdits produits d'hydrolyse comprenant les composants de membranes cellulaires sont soumis, avant filtration et/ou centrifugation, à un traitement thermique à une température allant de 60°C à 120°C jusqu'à dénaturation complète des composés thermolabiles.

22. Procédé selon l'une quelconque des revendications 14 à 19, **caractérisé en ce que** ledit surnageant des produits d'hydrolyse comprenant les composants de membranes cellulaires est soumis à un fractionnement au moyen d'une dialyse à travers une membrane semi-perméable et/ou une ultra filtration et/ou une filtration sur gel avec collecte d'une fraction comprenant les composants de faible masse moléculaire ayant une masse moléculaire M ≤ 10,0 kDa comme produit désiré.

23. Procédé selon l'une quelconque des revendications 14 à 19, **caractérisé en ce que** lesdits produits d'hydrolyse comprenant les composants de membranes cellulaires sont soumis à un fractionnement au moyen d'une dialyse à travers une membrane semi-perméable et/ou une ultra filtration et/ou une filtration sur gel avec collecte d'une fraction comprenant les composants de faible masse moléculaire ayant une masse moléculaire M ≤ 10,0 kDa comme produit désiré.

24. Procédé selon la revendication 20, **caractérisé en ce que** ledit surnageant des produits d'hydrolyse comprenant les composants de membranes cellulaires est soumis à un fractionnement au moyen d'une dialyse à travers une membrane semi-perméable et/ou une ultra filtration et/ou une filtration sur gel avec collecte d'une fraction comprenant les composants de faible masse moléculaire ayant une masse moléculaire M ≤ 10,0 kDa comme produit désiré.

**25.** Procédé selon la revendication 22, **caractérisé en ce que** ladite fraction comprenant les composants de faible masse moléculaire des membranes cellulaires est soumise à une chromatographie d'affinité sur des lectines avec collecte d'une fraction comprenant des composants contenant des hydrates de carbone comme produit désiré.

**26.** Procédé selon la revendication 24, **caractérisé en ce que** ladite fraction comprenant les composants de faible masse moléculaire des membranes cellulaires est soumise à une chromatographie d'affinité sur des lectines avec collecte d'une fraction comprenant des composants contenant des hydrates de carbone comme produit désiré.

**27.** Préparation pour la normalisation de l'état physiologique d'organismes humains et animaux, qui contient un agent biologiquement actif ayant des propriétés d'immunomodulation selon la revendication 1 dans une quantité de 0,001 à 85,0 pour cent en masse avec le reste étant un excipient.

**28.** Préparation selon la revendication 27, **caractérisée en ce que** ledit agent biologiquement actif comprend des composés organiques au taux qui suit en pour cent en masse :

| | |
|---|---|
| Peptides | de 28,0 à 60,0 ; |
| Acides aminés | de 0,5 à 31,5 ; |
| Hydrates de carbone | de 16,0 à 70,0 ; |
| Lipides, nucléotides et autres composés organiques | de 0,5 à 17,0. |

**29.** Préparation selon la revendication 28, **caractérisée en ce que** ledit agent biologiquement actif comprend des composés organiques au taux qui suit en pour cent en masse :

| | |
|---|---|
| Peptides | de 40,0 à 60,0 ; |
| Acides aminés | de 12,9 à 17,1 ; |
| Hydrates de carbone | de 16,0 à 32,8 ; |
| Lipides, nucléotides et autres composés organiques | de 4,4 à 10,6. |

**30.** Préparation selon la revendication 28, **caractérisée en ce que** ledit agent biologiquement actif comprend des composés organiques au taux qui suit en pour cent en masse :

| | |
|---|---|
| Peptides | de 34,0 à 50,1 ; |
| Acides aminés | de 16,6 à 22,6 ; |
| Hydrates de carbone | de 22,7 à 34,2 ; |
| Lipides, nucléotides et autres composés organiques | de 5,4 à 10,6. |

**31.** Préparation selon la revendication 28, **caractérisée en ce que** ledit agent biologiquement actif comprend des composés organiques au taux qui suit en pour cent en masse :

| | |
|---|---|
| Peptides | de 26,0 à 33,6 ; |
| Acides aminés | de 16,5 à 31,5 ; |
| Hydrates de carbone | de 20,9 à 37,9 ; |
| Lipides, nucléotides et autres composés organiques | de 8,6 à 17,0. |

**32.** Préparation selon la revendication 28, **caractérisée en ce que** ledit agent biologiquement actif comprend des composés organiques au taux qui suit en pour cent en masse :

| | |
|---|---|
| Peptides | de 29,0 à 36,3 ; |
| Acides aminés | moins de 0,5 ; |

(suite)

| Hydrates de carbone | de 57,7 à 70,0 ; |
|---|---|
| Lipides, nucléotides et autres composés organiques | de 0,5 à 5,5. |

33. Préparation selon l'une quelconque des revendications 30 à 32, **caractérisée en ce que** ledit excipient est une solution physiologique de chlorure de sodium et/ou une solution tampon saline et/ou des composés d'hydrates de carbone et/ou des graisses de minéraux, d'animaux, de végétaux ou d'origine synthétique ou une quelconque combinaison de ceux-ci.

34. Préparation selon l'une quelconque des revendications 28 à 33, **caractérisée en ce qu'**elle contient en outre un conservateur.

35. Utilisation d'un agent biologiquement actif ayant des propriétés d'immunomodulation selon la revendication 1 pour la fabrication d'une préparation pour la normalisation de l'état physiologique d'organismes humains et animaux, ladite préparation étant appropriée pour une administration auxdits organismes et consistant en ledit agent biologiquement actif dans une quantité allant de 0,001 à 85,0 pour cent en masse avec le reste étant un excipient.

36. Utilisation selon la revendication 35, **caractérisée en ce que** ladite préparation étant appropriée pour une administration comme injections intramusculaires à une dose allant de 0,005 à 0,5 mg/kg du poids corporel avec des intervalles de 1 à 7 jours entre les injections et/ou inhalations à une dose allant de 0,012 à 0,12 mg/kg du poids corporel avec des intervalles de 4 à 48 heures entre les inhalations et/ou administrations sublinguales à une dose allant de 0,012 à 0,12 mg/kg du poids corporel avec des intervalles de 4 à 48 heures entre les administrations et/ou des alimentations sous forme liquide à une dose allant de 0,02 à 1,0 mg/kg du poids corporel avec des intervalles de 1 à 10 jours entre les alimentations et/ou des pilules orales à une dose allant de 0,02 à 1,0 mg/kg du poids corporel avec des intervalles de 1 à 10 jours entre les administrations et/ou administrations intranasales à une dose allant de 0,001 à 0,05 mg/kg du poids corporel avec des intervalles de 2 à 24 heures entre les administrations jusqu'à la normalisation des paramètres physiologiques.

37. Utilisation selon la revendication 35, **caractérisée en ce que** ladite préparation étant appropriée pour une administration comme applications sur les surfaces de muqueuses ou de plaies sous la forme de compresses, de suppositoires, d'onguents, de poudres et de gels avec des intervalles de 1 à 10 jours entre les applications jusqu'à la normalisation des paramètres physiologiques.